(19) Europäisches Patentamt
European Patent Office
Office européen des brevets

(11) EP 4 624 461 A1

(12) **EUROPEAN PATENT APPLICATION**
published in accordance with Art. 153(4) EPC

(43) Date of publication:
**01.10.2025 Bulletin 2025/40**

(21) Application number: **23894158.7**

(22) Date of filing: **16.06.2023**

(51) International Patent Classification (IPC):
***C07D 307/62*** *(2006.01)* ***A61K 8/67*** *(2006.01)*
***A61Q 19/02*** *(2006.01)*

(52) Cooperative Patent Classification (CPC):
**A61K 8/67; A61Q 19/02; C07D 307/62**

(86) International application number:
**PCT/JP2023/022484**

(87) International publication number:
**WO 2024/111142 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.11.2022 JP 2022188247**
**11.04.2023 JP 2023064299**

(71) Applicant: **Seiwa Kasei Company, Limited**
**Higashiosaka-shi, Osaka 579-8004 (JP)**

(72) Inventors:
- **YOSHIOKA, Masato**
**Higashiosaka-shi, Osaka 579-8004 (JP)**
- **HONDA, Tasuku**
**Higashiosaka-shi, Osaka 579-8004 (JP)**
- **ITO, Masahiro**
**Higashiosaka-shi, Osaka 579-8004 (JP)**
- **IWAKI, Nobuyasu**
**Higashiosaka-shi, Osaka 579-8004 (JP)**
- **TOMIYAMA, Ai**
**Higashiosaka-shi, Osaka 579-8004 (JP)**
- **SUZUKI, Saki**
**Higashiosaka-shi, Osaka 579-8004 (JP)**

(74) Representative: **Vossius & Partner**
**Patentanwälte Rechtsanwälte mbB**
**Siebertstrasse 3**
**81675 München (DE)**

# (54) ASCORBIC ACID DERIVATIVE OR SALT THEREOF, AND COSMETIC THEREOF

(57) As a novel ascorbic acid derivative or a salt thereof that has the superior inherent functions of ascorbic acid such as a whitening action, a collagen production promoting action, and a moisture retaining action, while also being stable even when stored for a long period, demonstrating little change in color, change in smell, reduction in activity, and similar, the present invention provides an ascorbic acid derivative or a salt thereof wherein one hydrogen among the hydroxyl groups in the second position and the third position of ascorbic acid has been substituted with $R-O-CH_2-CH(OH)-CH_2-$, $R-O-CH_2-CH(CH_2OH)-$, $R-CH(CH_2OH)-$, $R-CH(OH)-CH_2-$ (R being H, an alkyl group, an alkenyl group, or a phenyl group), $HO-C(CH_3)_2-CH_2-$, or $HO-CH_2-C(CH_3)_2-$. The present invention also provides a cosmetic including said ascorbic acid derivative or a salt thereof.

EP 4 624 461 A1

## Description

### TECHNICAL FIELD

The present invention relates to an ascorbic acid derivative or a salt thereof that is suitable for use as a raw material for cosmetics, and further relates to a cosmetic containing the ascorbic acid derivative or a salt thereof.

### BACKGROUND ART

Ascorbic acid is a safe and useful antioxidant and is known as a compound having excellent whitening effects. On the other hand, it is unstable to light, heat, and oxidation, which has hindered its use in the field of cosmetics. Therefore, various ascorbic acid derivatives or salts thereof have been proposed as having improved stability over time compared to ascorbic acid, and their incorporation into external skin preparations for whitening (Patent document 1, Patent document 2) and into cosmetics (Patent document 3) have been proposed.
However, many of the above-mentioned ascorbic acid derivatives and salts thereof have problems such as discoloration and odor generation over time, and their stability over time is still insufficient. In addition, their activity in the body only lasts for a short period of time, and improvements in these areas are desired.
The present inventors have proposed an ascorbic acid derivative that solves these problems (Patent document 4), but further improvements are desired.

### PRIOR ART DOCUMENT

#### Patent Document

Patent Document 1: Japanese Patent Application Laid-Open No. 62-221611
Patent Document 2: JP 2005-060239 A
Patent Document 3: Japanese Patent Application Laid-open No. 1-228978
Patent Document 4: JP 4681670 B

### SUMMARY OF THE INVENTION

#### PROBLEM TO BE SOLVED BY THE INVENTION

An object of the present invention is to provide an ascorbic acid derivative or a salt thereof which has the excellent effects inherent to ascorbic acid, such as whitening effect and moisturizing effect, and is highly stable with little discoloration, odor generation, decrease in activity, etc. over time, and further has excellent physiological activity effects, and to provide a cosmetic preparation containing the same.

#### MEANS FOR SOLVING THE PROBLEM

The present inventors have conducted extensive studies in light of the above-mentioned circumstances, and have found that a novel ascorbic acid derivative or salt thereof represented by the following formula (I) has excellent effects such as whitening and moisturizing, is stable with little discoloration, odor generation, decrease in activity, etc. over time, and has excellent physiological activity effects such as hyaluronic acid production promoting effect. The present invention has been completed based on these findings.
The present invention provides an ascorbic acid derivative or a salt thereof, characterized by being represented by the following general formula (I) (claim 1).

[Chemical formula 1]

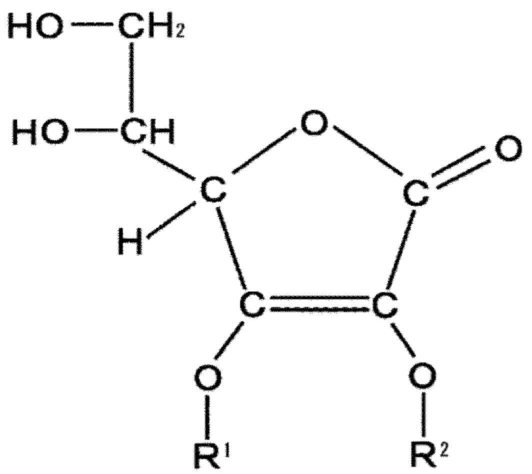

(I)

[wherein $R^1$ and $R^2$ are H, an alkyl group having 1 to 22 carbon atoms, an alkenyl group having 2 to 22 carbon atoms, a benzyl group, $R^3$ -O-$CH_2$ - CH(OH)-$CH_2$-, $R^3$-O-$CH_2$ -CH($CH_2$OH)-, $R^3$-CH($CH_2$OH)-, $R^3$-CH(OH)-$CH_2$-, HO-C$(CH_3)_2$-$CH_2$-, or HO-$CH_2$-C$(CH_3)_2$-, and $R^3$ is H, an alkyl group having 1 to 22 carbon atoms, an alkenyl group having 2 to 22 carbon atoms, or a phenyl group, provided that at least one of $R^1$ and $R^2$ is HO-C$(CH_3)_2$ -$CH_2$ - or HO-$CH_2$-C$(CH_3)_2$-.]

The salt of the ascorbic acid derivative means a compound in which H in the ascorbic acid derivative represented by formula (I) where $R^1$ or $R^2$ is H is replaced by a cation such as a metal ion or an ammonium ion. This salt is also included in the present invention.

The ascorbic acid derivative represented by the general formula (I) or salt thereof has superior stability over time compared to ascorbic acid or conventional ascorbic acid derivatives, maintains a high residual rate even when stored for several weeks in a high temperature environment above room temperature, and suppresses the problems of odor generation and coloration. In addition, it has superior antioxidant effect compared to conventional ascorbic acid derivatives such as glyceryl ascorbic acid. Among them, an ascorbic acid derivative having a hydroxyisobutyl group at the 3-position or salt thereof is preferred.

Ascorbic acid derivatives represented by general formula (I), in which either $R^1$ or $R^2$ is H, can be obtained by reacting ascorbic acid with isobutylene oxide or a halogenated tert-butyl alcohol, and regiospecifically etherifying only the 2-position and/or the 3-position among the four hydroxyl groups at the 2-position, 3-position, 5-position and 6-position of ascorbic acid with HO-C$(CH_3)_2$-$CH_2$- or HO-$CH_2$-C$(CH_3)_2$-, as described below.

In the reaction of ascorbic acid with isobutylene oxide or halogenated tert-butyl alcohol, depending on the difference in the etherification reaction conditions, the hydroxyl group at 2-position may be mainly etherified, or the hydroxyl group at 3-position may be mainly etherified. When the hydroxyl group at 2-position is mainly etherified, a mixture of $R^1$ being HO-C$(CH_3)_2$-$CH_2$- and $R^1$ being HO-$CH_2$-C$(CH_3)_2$ - may be produced. When the hydroxyl group at 3-position is mainly etherified, a mixture of $R^2$ being HO-C$(CH_3)_2$-$CH_2$- and $R^2$ being HO-$CH_2$-C$(CH_3)_2$- may be produced.

Among the ascorbic acid derivatives represented by general formula (I), those in which $R^1$ in formula (I) is H and $R^2$ is HO-C$(CH_3)_2$-$CH_2$- or HO-$CH_2$ -C$(CH_3)_2$- have superior melanin production inhibitory effects and collagen production promoting effects compared to conventional ascorbic acid derivatives such as glyceryl ascorbic acid.

Among the ascorbic acid derivatives represented by general formula (I), those in which one of $R^1$ and $R^2$ in formula (I) is HO-C$(CH_3)_2$-$CH_2$- or HO-$CH_2$ -C$(CH_3)_2$- and the other is an alkyl group having 1 to 22 carbon atoms, an alkenyl group having 2 to 22 carbon atoms, a benzyl group, $R^3$-O-$CH_2$-CH(OH)-$CH_2$-, $R^3$-O-$CH_2$ -CH($CH_2$ OH)-, $R^3$-CH($CH_2$OH)-, or $R^3$ -CH(OH)-$CH_2$-, and those in which both $R^1$ and $R^2$ are HO-C$(CH_3)_2$-$CH_2$- or HO-$CH_2$-C$(CH_3)_2$- have superior melanin production inhibitory effects, collagen production promoting effects, and hyaluronic acid production promoting effects, as compared with conventional ascorbic acid derivatives such as glyceryl ascorbic acid.

Among the ascorbic acid derivatives represented by general formula (I), those in which one of $R^1$ and $R^2$ in formula (I) is HO-C$(CH_3)_2$-$CH_2$- or HO-$CH_2$-C$(CH_3)_2$- and the other is an alkyl group having 1 to 22 carbon atoms, an alkenyl group having 2 to 22 carbon atoms, a benzyl group, $R^3$-O-$CH_2$-CH(OH)-$CH_2$-, $R^3$-O-$CH_2$-CH($CH_2$OH)-, $R^3$-CH($CH_2$OH)-, or $R^3$-CH(OH)-$CH_2$-can be obtained by reacting a compound in which one of $R^1$ and $R^2$ is HO-C$(CH_3)_2$-$CH_2$- or HO-$CH_2$-C$(CH_3)_2$- obtained by reacting ascorbic acid with isobutylene oxide or halogenated tert-butyl alcohol, with glycidol, an alkyl glycidyl ether of a specific structure, an alkenyl glycidyl ether, a phenyl glycidyl ether, a

sulfate ester, an alkylene oxide, an alkenyl oxide, a styrene oxide, an alkyl halide, a hydroxyalkyl halide, a benzyl halide, an alkenyl halide, a hydroxyalkenyl halide, a halogenated phenethyl alcohol, or the like; or can be obtained by reacting ascorbic acid with glycidol, an alkyl glycidyl ether of a specific structure, an alkenyl glycidyl ether, a phenyl glycidyl ether, a sulfate ester, an alkylene oxide, an alkenyl oxide, a styrene oxide, an alkyl halide, a hydroxyalkyl halide, a benzyl halide, an alkenyl halide, a hydroxyalkenyl halide, a halogenated phenethyl alcohol or the like, with either $R^1$ or $R^2$, and then, reacting the resulting compound with isobutylene oxide or a halogenated tert-butyl alcohol.

The invention according to claim 2 is the ascorbic acid derivative or a salt thereof according to claim 1, wherein, in the general formula (I), one of $R^1$ and $R^2$ is $HO\text{-}C(CH_3)_2\text{-}CH_2\text{-}$ or $HO\text{-}CH_2\text{-}C(CH_3)_2\text{-}$, and the other of $R^1$ and $R^2$ is an alkyl group having 2 to 20 carbon atoms or a branched alkylglyceryl group having 6 to 20 carbon atoms.

Among the ascorbic acid derivatives according to claim 1, those in which one of $R^1$ and $R^2$ is $HO\text{-}C(CH_3)_2\text{-}CH_2\text{-}$ or $HO\text{-}CH_2$ $\text{-}C(CH_3)_2\text{-}$ and the other is an alkyl group having 2 to 20 carbon atoms, preferably 6 to 16 carbon atoms, or a branched alkylglyceryl group having 6 to 20 carbon atoms, preferably 8 to 14 carbon atoms, are preferred in that they have a particularly high hyaluronic acid production promoting effect. Claim 2 corresponds to this particularly preferred embodiment.

The invention according to claim 3 is the ascorbic acid derivative or a salt thereof according to claim 1, wherein, in the general formula (I), one of $R^1$ and $R^2$ is $HO\text{-}C(CH_3)_2\text{-}CH_2\text{-}$ or $HO\text{-}CH_2$ $\text{-}C(CH_3)_2\text{-}$ and the other of $R^1$ and $R^2$ is a benzyl group.

Among the ascorbic acid derivatives according to claim 1, those in which one of $R^1$ and $R^2$ is $HO\text{-}C(CH_3)_2\text{-}CH_2\text{-}$ or $HO\text{-}CH_2$ $\text{-}C(CH_3)_2\text{-}$ and the other is a benzyl group are preferred in that they exhibit high physiologically active effects such as a hyaluronic acid production promoting effect and a melanin production inhibiting effect. Claim 3 corresponds to this particularly preferred embodiment.

The invention according to claim 4 is the ascorbic acid derivative or a salt thereof according to claim 1, wherein, in the general formula (I), one of $R^1$ and $R^2$ is $HO\text{-}C(CH_3)_2\text{-}CH_2\text{-}$ or $HO\text{-}CH_2$ $\text{-}C(CH_3)_2\text{-}$ and the other of $R^1$ and $R^2$ is $R^3\text{-}O\text{-}CH_2\text{-}CH(OH)\text{-}CH_2\text{-}$ or $R^3\text{-}O\text{-}CH_2\text{-}CH(CH_2OH)\text{-}$.

The ascorbic acid derivatives according to claim 1 have superior stability over time to ascorbic acid, and among them, those in which one of $R^1$ and $R^2$ is $HO\text{-}C(CH_3)_2\text{-}CH_2\text{-}$ or $HO\text{-}CH_2$ $\text{-}C(CH_3)_2\text{-}$ and the other is $R^3\text{-}O\text{-}CH_2\text{-}CH(OH)\text{-}CH_2\text{-}$ or $R^3\text{-}O\text{-}CH_2\text{-}CH(CH_2OH)\text{-}$ are preferred in that they have less odor generation among the ascorbic acid derivatives of the present invention. Claim 4 corresponds to this particularly preferred embodiment.

The invention according to claim 5 is the ascorbic acid derivative or a salt thereof according to claim 1, wherein, in the general formula (I), one of $R^1$ and $R^2$ is $HO\text{-}C(CH_3)_2\text{-}CH_2\text{-}$ or $HO\text{-}CH_2$ $\text{-}C(CH_3)_2\text{-}$ and the other of $R^1$ and $R^2$ is H, alkyl or alkenyl, and the total number of carbon atoms in $R^1$ and $R^2$ is 8 or less, or the other of $R^1$ and $R^2$ is $R^a\text{-}O\text{-}CH_2\text{-}CH(OH)\text{-}CH_2\text{-}$, $R^3\text{-}O\text{-}CH_2\text{-}CH(CH_2OH)\text{-}$, $HO\text{-}C(CH_3)_2\text{-}CH_2\text{-}$ or $HO\text{-}CH_2$ $\text{-}C(CH_3)_2\text{-}$, and the number of carbon atoms in $R^3$ is 4 or less.

The ascorbic acid derivative according to claim 1 has superior stability over time to ascorbic acid, and among them, those in which, in the general formula (I),
one of $R^1$ and $R^2$ is $HO\text{-}C(CH_3)_2\text{-}CH_2\text{-}$ or $HO\text{-}CH_2$ $\text{-}C(CH_3)_2\text{-}$ and the other of $R^1$ and $R^2$ is H, alkyl or alkenyl, and the total number of carbon atoms in $R^1$ and $R^2$ is 8 or less, or the other of $R^1$ and $R^2$ is $R^3\text{-}O\text{-}CH_2\text{-}CH(OH)\text{-}CH_2\text{-}$, $R^3\text{-}O\text{-}CH_2\text{-}CH(CH_2OH)\text{-}$, $HO\text{-}C(CH_3)_2\text{-}CH_2\text{-}$ or $HO\text{-}CH_2$ $\text{-}C(CH_3)_2\text{-}$, and the number of carbon atoms in $R^3$ is 4 or less are preferred in that they have a high moisturizing effect when incorporated into cosmetics. Claim 5 corresponds to this particularly preferred embodiment.

The invention according to claim 6 is the ascorbic acid derivative or a salt thereof according to claim 1, wherein, in the general formula (I), one of $R^1$ and $R^2$ is $HO\text{-}C(CH_3)_2\text{-}CH_2\text{-}$ or $HO\text{-}CH_2\text{-}C(CH_3)_2\text{-}$, and the other of $R^1$ and $R^2$ is $R^3\text{-}CH(CH_2OH)\text{-}$ or $R^3\text{-}CH(OH)\text{-}CH_2\text{-}$, and $R^3$ is H, an alkyl group having 1 to 20 carbon atoms, an alkenyl group having 2 to 20 carbon atoms, or a phenyl group.

The ascorbic acid derivatives according to claim 1 have superior stability over time to ascorbic acid, and among them, those in which , in the general formula (I), one of $R^1$ and $R^2$ is $HO\text{-}C(CH_3)_2\text{-}CH_2\text{-}$ or $HO\text{-}CH_2\text{-}C(CH_3)_2\text{-}$, and the other of $R^1$ and $R^2$ is $R^3\text{-}CH(CH_2OH)\text{-}$ or $R^3\text{-}CH(OH)\text{-}CH_2\text{-}$, and $R^3$ is H, an alkyl group having 1 to 20 carbon atoms, an alkenyl group having 2 to 20 carbon atoms, or a phenyl group are preferred in that coloring is suppressed among the ascorbic acid derivatives of the present invention. Claim 6 corresponds to this particularly preferred embodiment.

By incorporating the ascorbic acid derivative or salt thereof of the present invention into a cosmetic, it is possible to obtain a cosmetic that is excellent in whitening and moisturizing effects, is stable even when stored for a long period of time, and has high physiological activity. Therefore, the present invention provides a cosmetic that contains the ascorbic acid derivative or a salt thereof of the present invention (claim 7). Furthermore, since the effects of the present invention are particularly exhibited when the ascorbic acid derivative or salt thereof is incorporated in the cosmetic at 1 to 20% by mass, the present invention further provides a cosmetic in which the ascorbic acid derivative or a salt thereof of the present invention is incorporated in an amount of 1 to 20% by mass (claim 8).

EFFECT OF THE INVENTION

The ascorbic acid or salt thereof represented by the general formula (I) of the present invention has excellent functions inherent to ascorbic acid derivatives, such as whitening and moisturizing effects, is stable even after long-term storage with less discoloration, odor generation, and decrease in activity over time, and has high physiological activities, such as antioxidant effects, melanin production inhibitory effects, collagen production promoting effects, and hyaluronic acid production promoting effects. Therefore, by incorporating this compound into cosmetics such as skin external preparations and hair cosmetics, it is possible to obtain cosmetics that are excellent in whitening and moisturizing effects, stable even after long-term storage, and have high physiological activities. The cosmetics of the present invention are cosmetics (whitening cosmetics, moisturizing cosmetics, etc.) that are excellent in whitening and moisturizing effects, stable even after long-term storage, and have high physiological activities.

MODES FOR CARRYING OUT THE INVENTION

Hereinafter, $HO-C(CH_3)_2-CH_2-$ or $HO-CH_2-C(CH_3)_2-$ will be referred to as a "hydroxyisobutyl group". Hydroxyisobutyl ascorbic acid refers to ascorbic acid in which a hydroxyisobutyl group is bonded to any one or more of the oxygens of each hydroxyl group. The ascorbic acid derivative of the present invention is hydroxyisobutyl ascorbic acid in which a hydroxyisobutyl group is bonded to the 2-position and/or 3-position.
Specific examples of the ascorbic acid derivative represented by the general formula (I) include the compounds shown below, but the scope of the present invention is not limited to these.
In the following examples,

"Glyceryl" refers to $HOCH_2-CH(OH)-CH_2-$ or $HOCH_2-CH(CH_2OH)-$;
"Alkylglyceryl group" refers to $R-O-CH_2-CH(OH)CH_2-$ or $R-O-CH_2-CH(CH_2\ OH)-$ (R represents an alkyl group);
"Hydroxyalkyl group" refers to a 1-alkyl-2-hydroxyethyl group represented by $R-CH(OH)-CH_2-$ (R represents an alkyl group);
"Hydroxyalkenyl group" refers to a 1-alkenyl-2-hydroxyethyl group represented by $R-CH(OH)-CH_2-$ (R represents an alkenyl group);
"Alkyl group" refers to methyl group, ethyl group, propyl group, isopropyl group, butyl group, isobutyl group, pentyl group, hexyl group, ethylhexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, eicosyl group, behenyl group, or the like.

"Alkenyl group" refers to vinyl group, allyl group, butenyl group, isobutenyl group, crotyl group, octenyl group, decenyl group, dodecenyl group, and the like.

(1) 3-O-hydroxyisobutyl ascorbic acid

Examples include

3-O-hydroxyisobutyl-2-O-alkyl ascorbic acids, such as 3-O-hydroxyisobutyl-2-O-ethyl ascorbic acid, 3-O-hydroxyisobutyl-2-O-butyl ascorbic acid, 3-O-hydroxyisobutyl-2-O-hexyl ascorbic acid, 3-O-hydroxyisobutyl-2 -O-octyl ascorbic acid, 3-O-hydroxyisobutyl-2-O-decyl ascorbic acid, 3-O-hydroxyisobutyl-2-O-ethylhexyl ascorbic acid, 3-O-hydroxyisobutyl-2-O-dodecyl ascorbic acid, 3-O-hydroxyisobutyl-2-O-tetradecyl ascorbic acid, and 3-O-hydroxyisobutyl-2-O-hexadecylascorbic acid;
3-O-hydroxyisobutyl-2-O-alkenyl ascorbic acid, such as 3-O-hydroxyisobutyl-2-O-allyl ascorbic acid, 3-O-hydroxyisobutyl-2-O-octenyl ascorbic acid, and 3-O-hydroxyisobutyl-2-O-dodecenyl ascorbic acid;
3-O-hydroxyisobutyl-2-O-glyceryl ascorbic acid;
3-O-hydroxyisobutyl-2-O-alkyl glyceryl ascorbic acid, such as 3-O-hydroxyisobutyl-2-O-butyl glyceryl ascorbic acid, 3-O-hydroxyisobutyl-2-O-ethylhexyl glyceryl ascorbic acid, and 3-O-hydroxyisobutyl-2-O-dodecyl glyceryl ascorbic acid;
3-O-hydroxyisobutyl-2-O-alkenylglyceryl ascorbic acid, such as 3-O-hydroxyisobutyl-2-O-dodecenylglyceryl ascorbic acid;
3-O-hydroxyisobutyl-2-O-phenylglyceryl ascorbic acid;
3-O-hydroxyisobutyl-2-O-benzyl ascorbic acid;
3-O-hydroxyisobutyl-2-O-(2-hydroxyalkyl)ascorbic acid, such as 3-O-hydroxyisobutyl-2-O-(2-hydroxypropyl)ascorbic acid, 3-O-hydroxyisobutyl-2-O-(2-hydroxydecyl)ascorbic acid, 3-O-hydroxyisobutyl-2-O-(2-hydroxyhexadecyl) ascorbic acid, and 3-O-hydroxyisobutyl-2-O-(2-hydroxyethylbenzene)ascorbic acid; and
3-O-hydroxyisobutyl-2-O-(1-alkyl-2- hydroxyethyl )ascorbic acid, such as 3-O-hydroxyisobutyl-2-O-(1-methyl-2-hydroxyethyl)ascorbic acid, 3-O-hydroxyisobutyl-2-O-(1-ethyl-2-hydroxyethyl)ascorbic acid, 3-O-hydroxyisobutyl-2-O-(1-octyl-2-hydroxyethyl)ascorbic acid, 3-O-hydroxyisobutyl-2-O-(1-tetradecyl-2-hydroxyethyl)ascorbic acid, and 3-O-hydroxyisobutyl-2-O-(1-phenyl-2-hydroxyethyl)ascorbic acid.

(2) 2-O-hydroxyisobutyl ascorbic acid

Examples include

2-O-hydroxyisobutyl-3-O-alkyl ascorbic acids, such as 2-O-hydroxyisobutyl-3-O-ethyl ascorbic acid, 2-O-hydroxyisobutyl-3-O-butyl ascorbic acid, 2-O-hydroxyisobutyl-3-O-hexyl ascorbic acid, 2-O-hydroxyisobutyl-3-O-octyl ascorbic acid, 2-O-hydroxyisobutyl-3-O-decyl ascorbic acid, 2-O-hydroxyisobutyl-3-O-ethylhexyl ascorbic acid, 2-O-hydroxyisobutyl-3-O-dodecyl ascorbic acid, 2-O-hydroxyisobutyl-3-O-tetradecyl ascorbic acid, and 2-O-hydroxyisobutyl-3-O-hexadecylascorbic acid;
2-O-hydroxyisobutyl-3-O-alkenyl ascorbic acid, such as 2-O-hydroxyisobutyl-3-O-allyl ascorbic acid, 2-O-hydroxyisobutyl-3-O-octenyl ascorbic acid, and 2-O-hydroxyisobutyl-3-O-dodecenyl ascorbic acid;
2-O-hydroxyisobutyl-3-O-glyceryl ascorbic acid;
2-O-hydroxyisobutyl-3-O-alkyl glyceryl ascorbic acid, such as 2-O-hydroxyisobutyl-3-O-butyl glyceryl ascorbic acid, 2-O-hydroxyisobutyl-3-O-ethylhexyl glyceryl ascorbic acid, and 2-O-hydroxyisobutyl-3-O-dodecyl glyceryl ascorbic acid;
2-O-hydroxyisobutyl-3-O-alkenylglyceryl ascorbic acid, such as 2-O-hydroxyisobutyl-3-O-dodecenylglyceryl ascorbic acid;
2-O-hydroxyisobutyl-3-O-phenylglyceryl ascorbic acid;
2-O-hydroxyisobutyl-3-O-benzyl ascorbic acid;
2-O-hydroxyisobutyl-3-O-(2-hydroxyalkyl)ascorbic acid, such as 2-O-hydroxyisobutyl-3-O-(2-hydroxypropyl)ascorbic acid, 2-O-hydroxyisobutyl-3-O-(2-hydroxydecyl)ascorbic acid, 2-O-hydroxyisobutyl-3-O-(2-hydroxyhexadecyl) ascorbic acid, and 2-O-hydroxyisobutyl-3-O-(2-hydroxyethylbenzene)ascorbic acid;
2-O-hydroxyisobutyl-3-O-(1-alkyl-2-hydroxyethyl)ascorbic acid, such as 2-O-hydroxyisobutyl-3-O-(1-methyl-2-hydroxyethyl)ascorbic acid, 2-O-hydroxyisobutyl-3-O-(1-ethyl-2-hydroxyethyl)ascorbic acid, 2-O-hydroxyisobutyl-3-O-(1-octyl-2-hydroxyethyl)ascorbic acid, 2-O-hydroxyisobutyl-3-O-(1-tetradecyl-2-hydroxyethyl)ascorbic acid, and 2-O-hydroxyisobutyl-3-O-(1-phenyl-2-hydroxyethyl)ascorbic acid.

(3) 2,3-di-O-hydroxyisobutyl ascorbic acid.

The ascorbic acid derivative or salt thereof of the present invention can be produced by various methods. For example, a hydroxyisobutyl group is introduced into the oxygen atom bonded to the 2- or 3-position of ascorbic acid by reacting ascorbic acid with isobutylene oxide or a halogenated tert-butyl alcohol, thereby synthesizing 2-O- or 3-O-hydroxyisobutyl ascorbic acid. Thereafter, the other oxygen atom bonded to the 2- or 3-position is alkylated or alkenylated by a known method to obtain the ascorbic acid derivative of the present invention. The compound of the present invention may be obtained by first alkylating or alkenylating the oxygen atom bonded to the 2- or 3-position of ascorbic acid, and then reacting the reaction product with isobutylene oxide or a halogenated tert-butyl alcohol.

Examples of the compound for introducing a hydroxyisobutyl group in the present invention include, but are not limited to, isobutylene oxide and halogenated tert-butyl alcohol. Examples of the halogenated tert-butyl alcohol include fluorinated tert-butyl alcohol, chlorinated tert-butyl alcohol, and brominated tert-butyl alcohol.

In the reaction of ascorbic acid or hydroxyalkylated ascorbic acid with isobutylene oxide or halogenated tert-butyl alcohol, depending on the difference in the etherification reaction conditions, the hydroxyl group at 2-position may be mainly etherified, or the hydroxyl group at 3-position may be mainly etherified. When the hydroxyl group at 2-position is mainly etherified, a mixture of R1 being $HO-C(CH_3)_2-CH_2$ - and $R_1$ being $HO-CH_2-C(CH_3)_2$- may be produced, and when the hydroxyl group at 3-position is mainly etherified, a mixture of $R_2$ being $HO-C(CH_3)_2-CH_2$- and $R^2$ being $HO-CH_2-C(CH_3)_2$- may be produced.

The amount of isobutylene oxide or halogenated tert-butyl alcohol used in the present invention is not particularly limited, but is preferably 0.8 to 1.5 mol, more preferably 1.0 to 1.2 mol, per mol of ascorbic acid.

The reaction for introducing a hydroxyisobutyl group in the production of the present invention can be carried out in various solvents. Examples of the solvents include, without limitation, water, lower alcohols such as methanol, ethanol and isopropanol, dimethylsulfoxide (DMSO) , N,N-dimethylformamide (DMF) , N,N-dimethylacetamide (DMAc), dioxane, tetrahydrofuran (THF), N-methylpyrrolidone, and mixtures thereof. The reaction temperature is not particularly limited, but

is preferably in the range of 30 to 100°C, more preferably in the range of 50 to 90°C, and particularly preferably in the range of 60 to 90°C.

The pH of the reaction solvent is not particularly limited, but when an ascorbic acid derivative or a salt thereof in which the 2-position of the ascorbic acid structure is H is produced, acidic conditions are preferred, and a pH of 2 to 6 is particularly preferred. Whereas in the production of the ascorbic acid derivative or a salt thereof in which the 3-position of the ascorbic acid structure is H, alkaline conditions are preferred, and a pH of 8 to 11 is particularly preferred.

Examples of pH adjusters used during the reaction include lactic acid, citric acid, glycolic acid, succinic acid, tartaric acid, malic acid, gluconic acid, sodium hydroxide, potassium hydroxide, potassium carbonate, sodium carbonate, sodium hydrogen carbonate, ammonium hydrogen carbonate, and triethylamine.

The ascorbic acid derivative or a salt thereof of the present invention produced as described above can be purified by means of column chromatography using silica gel, column chromatography using a resin such as an ion exchange resin, treatment with activated carbon, extraction, distillation, crystallization, or the like.

The ascorbic acid derivative represented by the above general formula (I) in which $R^1$ or $R^2$ is H can form a salt by replacing the hydrogen ion dissociated from H with a cation such as a metal ion or an ammonium ion, and such salts are also included in the scope of the present invention. Examples of such salts include inorganic salts and organic salts, and examples of inorganic salts include salts of alkali metal such as sodium and potassium, salts of alkaline earth metal such as calcium and magnesium, and ammonium salts. Examples of organic salts include diethanolamine salts, triethanolamine salts, and basic amino acid salts. The salt can be formed by a method similar to that of known salt formation, such as a method of neutralizing an aqueous solution of an ascorbic acid derivative in which $R^1$ or $R^2$ is H with a basic substance.

The hydroxyisobutyl ascorbic acid synthesized as described above can be alkylated, alkenylated, benzylated, hydroxyalkylated, or the like with glycidol, alkyl glycidyl ethers of a specific structures, alkenyl glycidyl ethers, phenyl glycidyl ethers, sulfates, alkylene oxides, alkenyl oxides, styrene oxides, alkyl halides, hydroxyalkyl halides, benzyl alcohol halides, alkenyl halides, hydroxyalkenyl halides, phenethyl alcohol halides, or the like to obtain the ascorbic acid derivatives of claims 2, 3, 4, or 6.

The amount of glycidol, alkyl glycidyl ether, alkylene oxide, alkenyl oxide, styrene oxide, or the like used in alkylation, alkenylation, benzylation, phenylation, or the like is not particularly limited, but is preferably 0.8 to 2.0 moles per mole of hydroxyisobutyl ascorbic acid.

The reaction with glycidol, alkyl glycidyl ether, alkylene oxide, alkenyl oxide, styrene oxide, and the like can be carried out in the same solvent, at the same reaction temperature, and at the same pH as in the reaction for introducing a hydroxyisobutylene group, and the ascorbic acid derivative of the present invention can be synthesized by purifying the reaction product by means of column chromatography using silica gel, column chromatography using a resin such as an ion exchange resin, activated carbon treatment, extraction, distillation, crystallization, or the like.

In addition, even when first reacting the oxygen atom bonded to the 2- or 3-position of ascorbic acid with glycidol, an alkyl glycidyl ether, an alkylene oxide, an alkenyl oxide, a styrene oxide, or the like, and then introducing a hydroxyisobutyl group into the other oxygen atom, each reaction can be carried out under the same conditions as described above.

The ascorbic acid derivative or a salt thereof of the present invention can be suitably used as a component of various cosmetics such as skin external preparations and hair cosmetics, and can also be used as a food additive, feed, etc.

When the ascorbic acid derivative or a salt thereof of the present invention is compounded in various cosmetics, the compounding amount is preferably 1% to 20% by weight, and particularly preferably in the range of 3% to 10% by weight. If the compounding amount is less than 1% by weight, the effects of the ascorbic acid derivative or a salt thereof of the present invention, such as the whitening effect and the hyaluronic acid production promoting effect, are often not fully exhibited. On the other hand, if the compounding amount are often not expected, and there is a risk of damaging the formulation system.

In addition to these essential components, the cosmetic of the present invention may contain, as appropriate, commonly used components such as oily raw materials, surfactants, moisturizers, polymeric compounds, antioxidants, whitening agents, drugs, ultraviolet absorbers, sequestering agents, proteins, protein hydrolysates or derivatives thereof, amino acids or derivatives thereof, pH adjusters, preservatives, etc. The ascorbic acid derivative or a salt thereof of the present invention is also effective as a moisturizer, and other moisturizers may also be compounded into the cosmetic of the present invention.

Examples of oil-based raw materials, surfactants, other moisturizing agents, polymer compounds, antioxidants, other whitening agents, other drugs, ultraviolet absorbers, sequestering agents, proteins, protein hydrolysates or derivatives thereof, amino acids or derivatives thereof, pH adjusters, preservatives, etc. include those similar to those described in JP 2022-028125 A.

The cosmetic composition of the present invention may have any agent system, including a solution system, a solubilized system, an emulsion system, a gel system, a powder dispersion system, and a water-oil two-layer system, and can be produced by compounding the ascorbic acid derivative represented by the above general formula (I) or a salt

thereof with the above-mentioned optional compounding components according to the desired product.

EXAMPLES

Next, specific embodiments for carrying out the present invention will be described in detail with reference to Examples, but the scope of the present invention is not limited to the Examples. Prior to the Examples, examples of the production of the ascorbic acid derivatives of the present invention used in the Examples and Comparative Examples will be described as Synthesis Examples.

Synthesis Example 1 Synthesis of 2-O-(2-hydroxyisobutyl)ascorbic acid

In an eggplant flask, water (10.5 mL), DMF (5 mL), ascorbic acid (10.0 g), sodium hydroxide (2.50 g), and isobutylene oxide (4.50 g) were added, and the resulting mixture was stirred at 60°C for 4 hours. After the reaction was completed, 5 mol/L hydrochloric acid was added to make the pH acidic. Then, the mixture was concentrated under reduced pressure, and isopropanol was added, followed by filtration and concentration under reduced pressure. The resulting residue (10.1 g) was subjected to silica gel chromatography, eluted with a mixture of chloroform/methanol = 10/0 to 6/4, and concentrated under reduced pressure to obtain 2-O-(2-hydroxyisobutyl)ascorbic acid (8.83 g).

The resulting product was subjected to mass spectrometry, $^{1}H$-NMR and $^{13}C$-NMR measurements, and the measurement results confirmed that the product was 2-O-(2-hydroxyisobutyl)ascorbic acid represented by the following structural formula:

[Chemical formula 2]

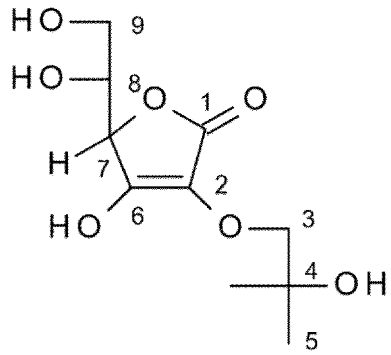

In this structural formula, the carbon atoms and hydrogen atoms bonded to the carbon atoms are omitted. For example, in this formula, the 1-, 2-, 4-, 6-, and 7-positions are carbons, the 5-position is a $CH_3$ group, the 3- and 9-positions are $CH_2$ groups, and the 8-position is a CH group. In the following structural formulas, hydrogen atoms and carbon atoms are omitted in the same manner as in this formula.

In the following synthesis examples, the products obtained were also subjected to mass spectrometry, $^{1}H$-NMR, and $^{13}C$-NMR measurements, and the measurement results confirmed that each product was an ascorbic acid derivative represented by the structural formula or compound name shown in each synthesis example. The measurement results of mass spectrometry, $^{1}H$-NMR, and $^{13}C$-NMR measurements for the products obtained in the synthesis examples are shown in Tables 1 to 8.

Synthesis Example 2 Synthesis of 3-O-(2-hydroxyisobutyl) ascorbic acid

According to the same manner as in Synthesis Example 1, except that DMF (50 mL) were used instead of water, DMF, and triethylamine (1.72 g) was used instead of sodium hydroxide, 3-O-(2-hydroxyisobutyl) ascorbic acid (7.42 g) in the title was obtained.

[Chemical formula 3]

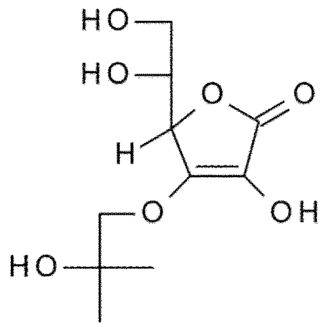

Synthesis Example 3 Synthesis of 2-O-(2-hydroxyisobutyl)-3-O-ethylascorbic acid

In an eggplant flask, 2-O-(2-hydroxyisobutyl)ascorbic acid (1.50 g) obtained in Synthesis Example 1, DMF (6 mL), triethylamine (1.22 g), and diethyl sulfate (1.86 g) were added, and the mixture was stirred at 70°C for 4 hours. After the reaction was completed, ion-exchanged water and ethyl acetate were added, and a separation operation of the mixture was conducted. The ethyl acetate layer was collected, and the extract was dried over anhydrous sodium sulfate and then concentrated under reduced pressure. The resulting residue (1.16 g) was subjected to silica gel chromatography, eluted with a mixture of chloroform/methanol=10/0 to 7/3, and concentrated under reduced pressure to obtain 2-O-(2-hydroxyisobutyl)-3-O-ethylascorbic acid (148.5 mg) represented by the following structural formula.

[Chemical formula 4]

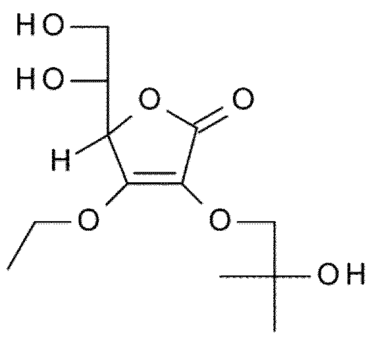

Synthesis Example 4 Synthesis of 2-O-(2-hydroxyisobutyl)-3-O-butylascorbic acid

According to the same manner as in Synthesis Example 3, except that sodium hydrogen carbonate (1.27 g) was used instead of triethylamine and butyl bromide (1.08 g) was used instead of diethyl sulfate, 2-O-(2-hydroxyisobutyl)-3-O-butylascorbic acid (710.3 mg) in the title was obtained.

[Chemical formula 5]

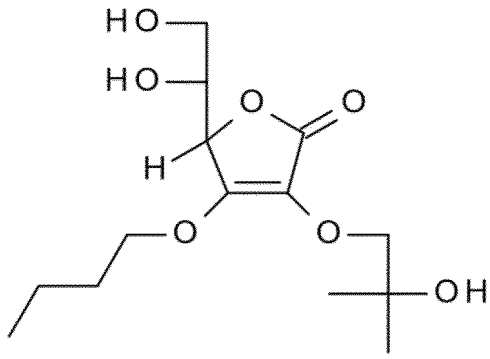

[0065]

Synthesis Example 5 Synthesis of 2-O-(2-hydroxyisobutyl)-3-O-hexyl ascorbic acid

According to the same manner as in Synthesis Example 3, except that sodium hydrogen carbonate (1.27 g) was used instead of triethylamine and hexyl bromide (1.30 g) was used instead of diethyl sulfate, 2-O-(2-hydroxyisobutyl)-3-O-hexyl ascorbic acid (473.7 mg) in the title was obtained.

[Chemical formula 6]

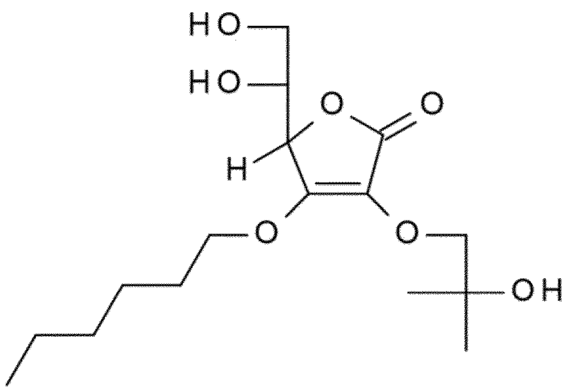

Synthesis Example 6 Synthesis of 2-O-(2-hydroxyisobutyl)-3-O-octyl ascorbic acid

According to the same manner as in Synthesis Example 3, except that sodium hydrogen carbonate (1.52 g) was used instead of triethylamine and octyl bromide (1.75 g) was used instead of diethyl sulfate, 2-O-(2-hydroxyisobutyl)-3-O-octyl ascorbic acid (148.5 mg) in the title was obtained.

[Chemical formula 7]

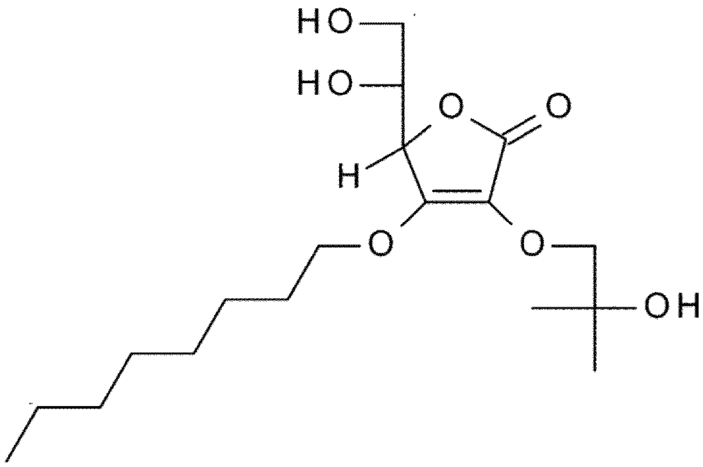

Synthesis Example 7 Synthesis of 2-O-(2-hydroxyisobutyl)-3-O-decyl ascorbic acid

According to the same manner as in Synthesis Example 3, except that sodium hydrogen carbonate (1.27 g) was used instead of triethylamine and decyl bromide (1.74 g) was used instead of diethyl sulfate, 2-O-(2-hydroxyisobutyl)-3-O-decyl ascorbic acid (371.2 mg) in the title was obtained.

[Chemical formula 8]

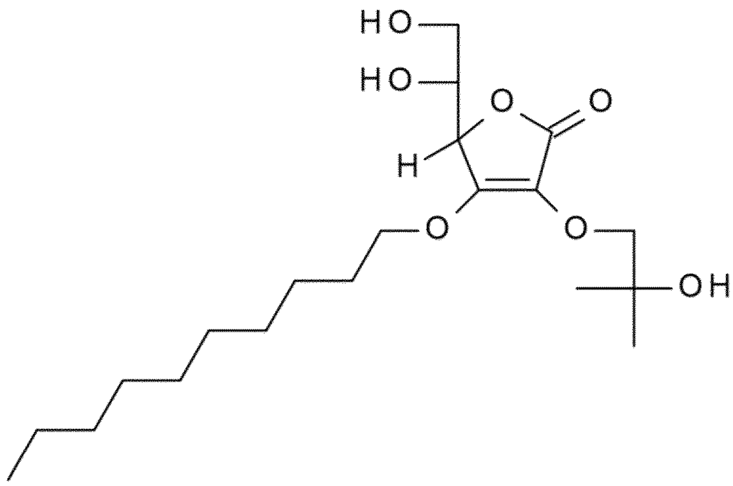

Synthesis Example 8 Synthesis of 2-O-(2-hydroxyisobutyl)-3-O-dodecyl ascorbic acid

According to the same manner as in Synthesis Example 3, except that sodium hydrogen carbonate (1.27 g) was used instead of triethylamine and dodecyl bromide (1.96 g) was used instead of diethyl sulfate, 2-O-(2-hydroxyisobutyl)-3-O-dodecyl ascorbic acid (358.8 mg) in the title was obtained.

[Chemical formula 9]

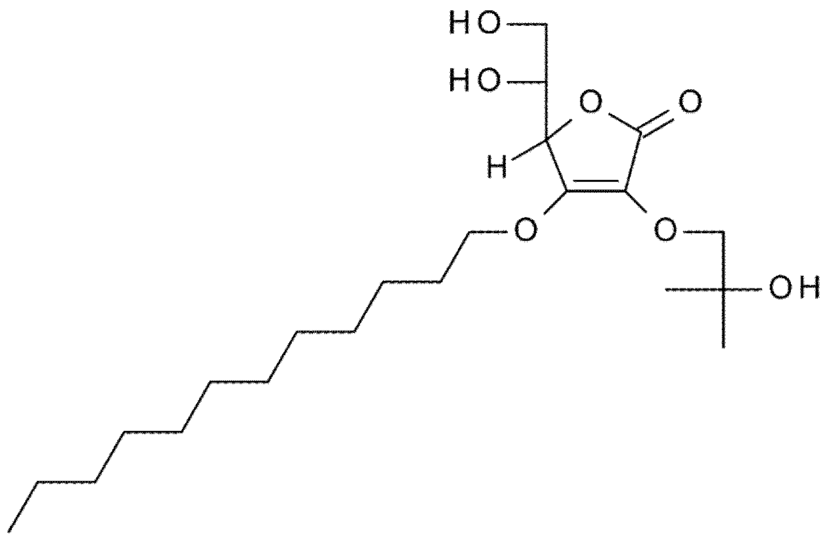

Synthesis Example 9 Synthesis of 2-O-(2-hydroxyisobutyl)-3-O-tetradecyl ascorbic acid

According to the same manner as in Synthesis Example 3, except that sodium hydrogen carbonate (1.27 g) was used instead of triethylamine and tetradecyl bromide (2.18 g) was used instead of diethyl sulfate, 2-O-(2-hydroxyisobutyl)-3-O-tetradecyl ascorbic acid (328.8 mg) in the title was obtained.

[Chemical formula 10]

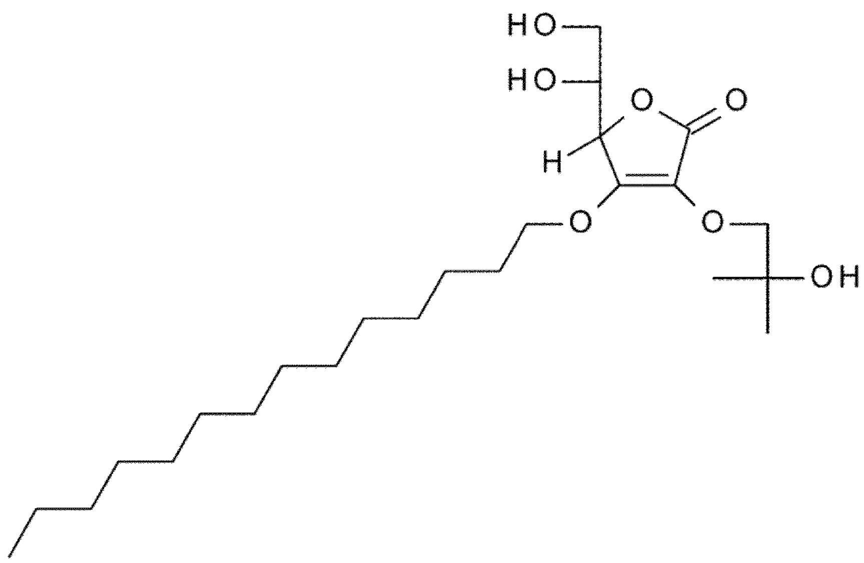

Synthesis Example 10: Synthesis of 2-O-(2-hydroxyisobutyl)-3-O-hexadecylascorbic acid

According to the same manner as in Synthesis Example 3, except that sodium hydrogen carbonate (1.27 g) was used instead of triethylamine and cetyl bromide (1.85 g) was used instead of diethyl sulfate, 2-O-(2-hydroxyisobutyl)-3-O-hexadecylascorbic acid (258.0 mg) in the title was obtained.

[Chemical formula 11]

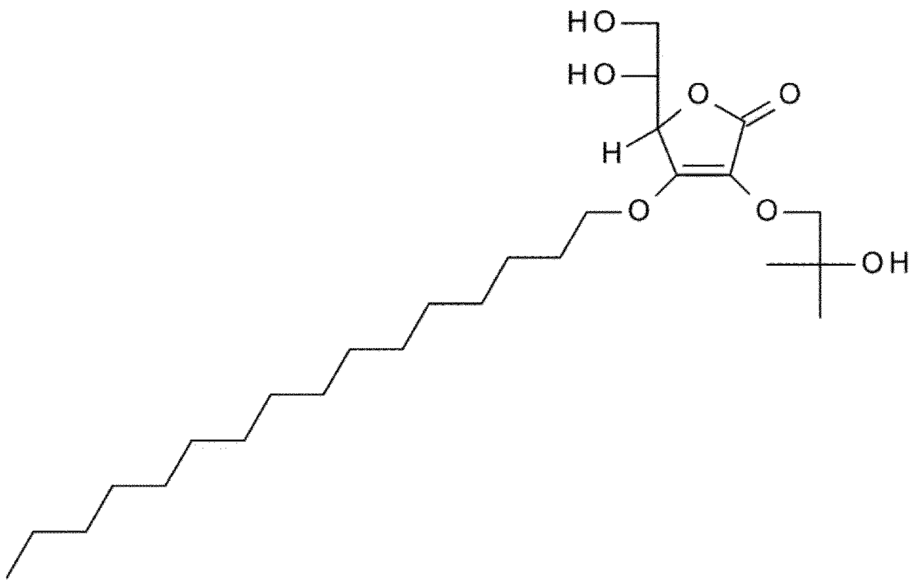

Synthesis Example 11 Synthesis of 2-O-(2-hydroxyisobutyl)-3-O-allylascorbic acid

According to the same manner as in Synthesis Example 3, except that sodium hydrogen carbonate (1.52 g) was used instead of triethylamine and allyl bromide (1.10 g) was used instead of diethyl sulfate, 2-O-(2-hydroxyisobutyl)-3-O-allylascorbic acid (366.4 mg) in the title was obtained.

[Chemical formula 12]

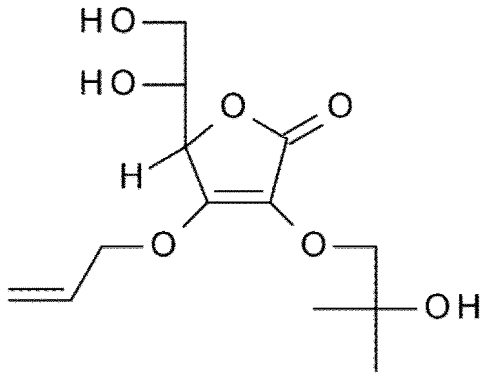

Synthesis Example 12 Synthesis of 2-O-(2-hydroxyisobutyl)-3-O-benzyl ascorbic acid

According to the same manner as in Synthesis Example 3, except that sodium hydrogen carbonate (1.52 g) was used instead of triethylamine and benzyl bromide (1.55 g) was used instead of diethyl sulfate, 2-O-(2-hydroxyisobutyl)-3-O-benzyl ascorbic acid (602.2 mg) in the title was obtained.

[Chemical formula 13]

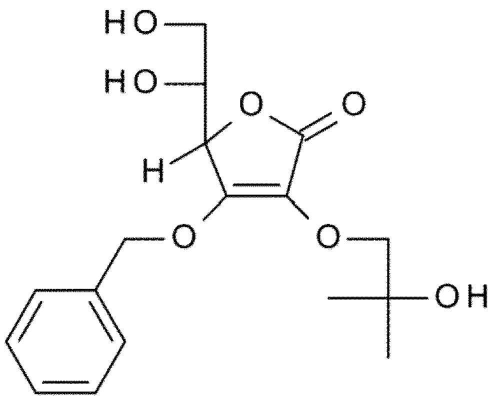

Synthesis Example 13: Synthesis of 2-O-(2-hydroxyisobutyl)-3-O-glyceryl ascorbic acid

In an eggplant flask, 3-O-glyceryl ascorbic acid (3.00 g), DMF (12 mL), triethylamine (0.24 g), and isobutylene oxide (1.04g) were added, and the mixture was stirred at 85°C for 18 hours. After the reaction was completed, ion-exchanged water and ethyl acetate were added, and a separation operation of the mixture was conducted. The ethyl acetate layer was collected, and the extract was dried over anhydrous sodium sulfate and then concentrated under reduced pressure. Resulting residue (1.12 g) was subjected to silica gel chromatography, eluted with a mixture of chloroform/methanol = 9.5/0.5 to 7/3, and concentrated under reduced pressure to obtain 2-O-(2-hydroxyisobutyl)-3-O-glyceryl ascorbic acid (178.6 mg) represented by the following structural formula.

[Chemical formula 14]

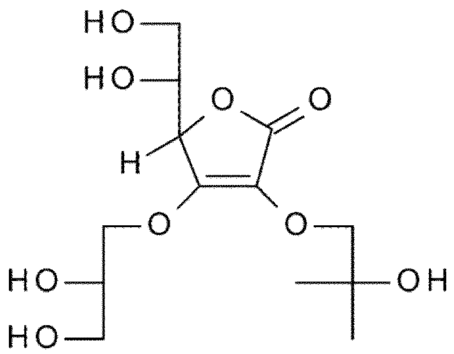

Synthesis Example 14: Synthesis of 2-O-(2-hydroxyisobutyl)-3-O-(2-hydroxy-3-O-butyl) ascorbic acid

According to the same manner as in Synthesis example 3, except that sodium hydrogen carbonate (0.20 g) was used instead of triethylamine and butyl glycidyl ether (1.89 g) was used instead of diethyl sulfate, 2-O-(2-hydroxyisobutyl)-3-O-(2-hydroxy-3-O-butyl)ascorbic acid (216.6 mg) in the title was obtained

[Chemical formula 15]

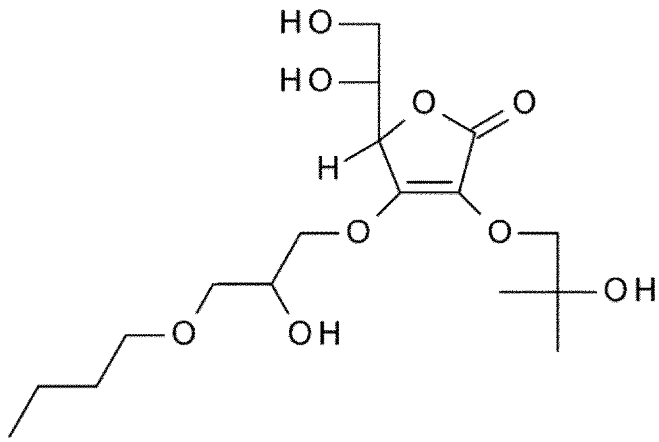

Synthesis Example 15: Synthesis of 2-O-(2-hydroxyisobutyl)-3-O-(2-hydroxy-3-O-ethylhexyl) ascorbic acid

According to the same manner as in Synthesis example 3, except that sodium hydrogen carbonate (0.20 g) was used instead of triethylamine and ethylhexyl glycidyl ether (2.70 g) was used instead of diethyl sulfate, 2-O-(2-hydroxyisobutyl)-3-O-(2-hydroxy-3-O-ethylhexyl)ascorbic acid (375.9 mg) in the title was obtained.

[Chemical formula 16]

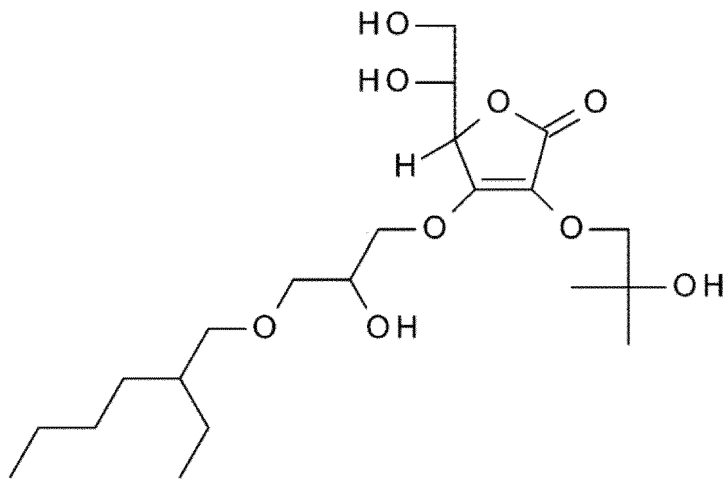

Synthesis Example 16: Synthesis of 2-O-(2-hydroxyisobutyl)-3-O-(2-hydroxy-3-O-dodecyl) ascorbic acid

According to the same manner as in Synthesis example 3, except that sodium hydrogen carbonate (0.20 g) was used instead of triethylamine and lauryl glycidyl ether (3.52 g) was used instead of diethyl sulfate, 2-O-(2-hydroxyisobutyl)-3-O-(2-hydroxy-3-O-dodecyl) ascorbic acid (450.3 mg) in the title was obtained.

[Chemical formula 17]

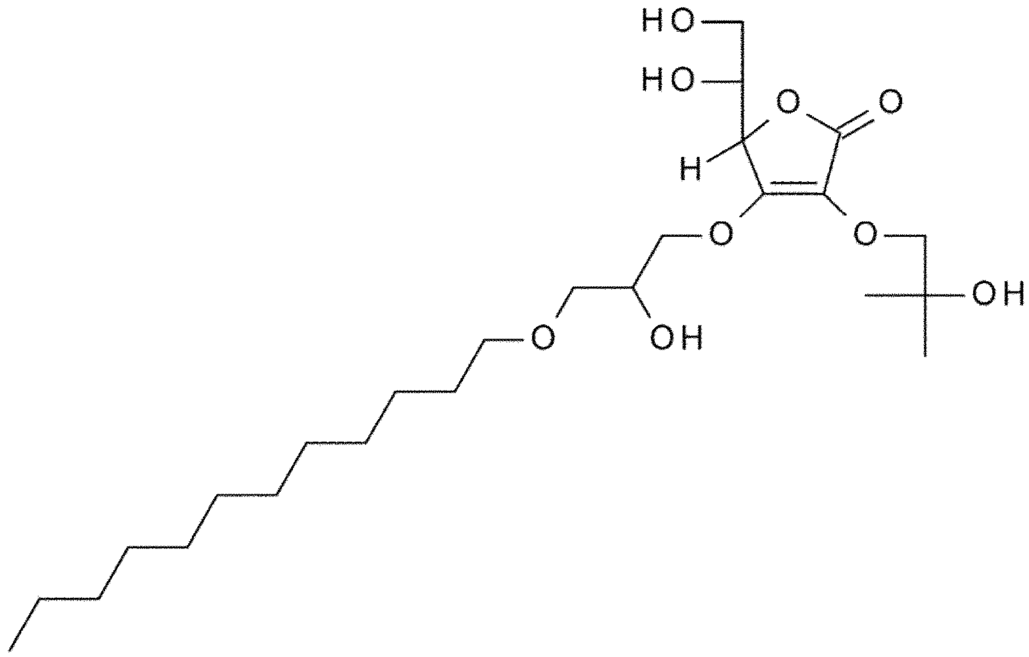

Synthesis Example 17 Synthesis of 2,3-di-O-(2-hydroxyisobutyl)ascorbic acid

According to the same manner as in Synthesis Example 3, except that isobutylene oxide (1.05 g) was used instead of diethyl sulfate, 2,3-di-O-(2-hydroxyisobutyl) ascorbic acid (500.9 mg) in the title was obtained.

[Chemical formula 18]

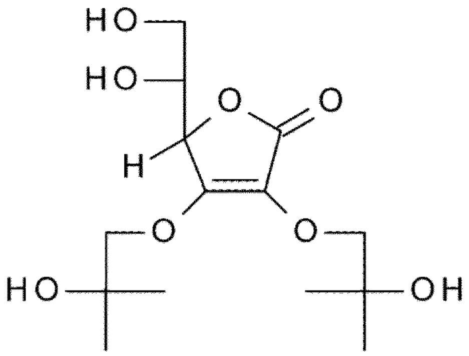

Synthesis Example 18 Synthesis of 2-O-ethyl-3-O-(2-hydroxyisobutyl) ascorbic acid

In an eggplant flask, 3-O-(2-hydroxyisobutyl)ascorbic acid (3.00 g) obtained in Synthesis Example 2, DMF (12 mL), triethylamine (1.35 g), and diethyl sulfate (2.42 g) were added, and the mixture was stirred at 80°C for 5 hours. After the reaction was completed, ion-exchanged water and ethyl acetate were added, and a separation operation of the mixture was conducted. The ethyl acetate layer was collected, and the extract was dried over anhydrous sodium sulfate and then concentrated under reduced pressure. The resulting residue (2.41 g) was subjected to silica gel chromatography, eluted with a mixture of chloroform/methanol = 10/0 to 9/1, and concentrated under reduced pressure to obtain 2-O-ethyl-3-O-(2-hydroxyisobutyl) ascorbic acid (443.0 mg) represented by the following structural formula.

[Chemical formula 19]

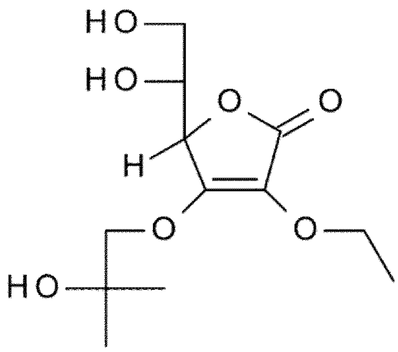

Synthesis Example 19: Synthesis of 2-O-butyl-3-O-(2-hydroxyisobutyl) ascorbic acid

According to the same manner as in Synthesis Example 18, except that sodium hydrogen carbonate (0.56 g) was used instead of triethylamine and butyl bromide (0.99 g) was used instead of diethyl sulfate, 2-O-butyl-3-O-(2-hydroxyisobutyl) ascorbic acid (464.8 mg) in the title was obtained.

[Chemical formula 20]

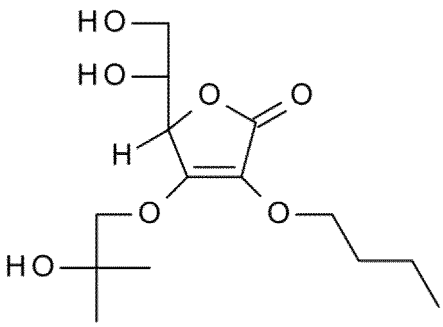

Synthesis Example 20: Synthesis of 2-O-hexyl-3-O-(2-hydroxyisobutyl) ascorbic acid

According to the same manner as in Synthesis Example 18, except that sodium hydrogen carbonate (0.56 g) was used instead of triethylamine and hexyl bromide (1.20 g) was used instead of diethyl sulfate, 2-O-hexyl-3-O-(2-hydroxyisobutyl) ascorbic acid (617.8 mg) in the title was obtained.

[Chemical formula 21]

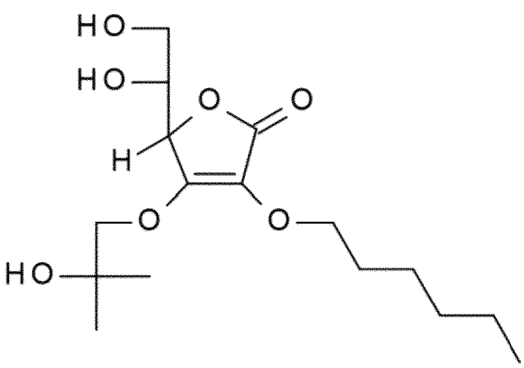

Synthesis Example 21 Synthesis of 2-O-octyl-3-O-(2-hydroxyisobutyl) ascorbic acid

According to the same manner as in Synthesis Example 18, except that sodium hydrogen carbonate (1.12 g) was used instead of triethylamine and octyl bromide (2.80 g) was used instead of diethyl sulfate, 2-O-octyl-3-O-(2-hydroxyisobutyl) ascorbic acid (1.05 g) in the title was obtained.

[Chemical formula 22]

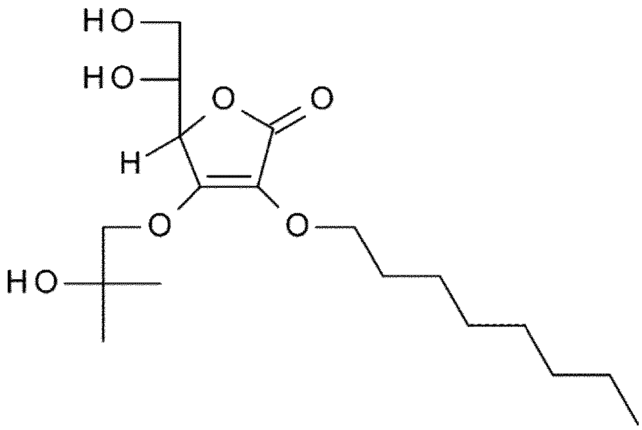

Synthesis Example 22 Synthesis of 2-O-decyl-3-O-(2-hydroxyisobutyl) ascorbic acid

According to the same manner as in Synthesis Example 18, except that sodium hydrogen carbonate (0.56 g) was used instead of triethylamine and decyl bromide (1.60 g) was used instead of diethyl sulfate, 2-O-decyl-3-O-(2-hydroxyisobutyl) ascorbic acid (216.7 mg) in the title was obtained.

[Chemical formula 23]

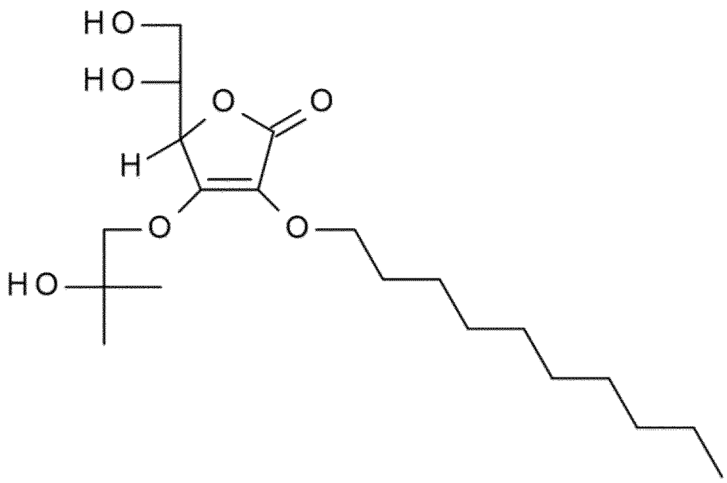

Synthesis Example 23: Synthesis of 2-O-dodecyl-3-O-(2-hydroxyisobutyl) ascorbic acid

According to the same manner as in Synthesis Example 18, except that sodium hydrogen carbonate (0.56 g) was used instead of triethylamine and dodecyl bromide (1.81 g) was used instead of diethyl sulfate, 2-O-dodecyl-3-O-(2-hydroxyisobutyl) ascorbic acid (248.9 mg) in the title was obtained.

[Chemical formula 24]

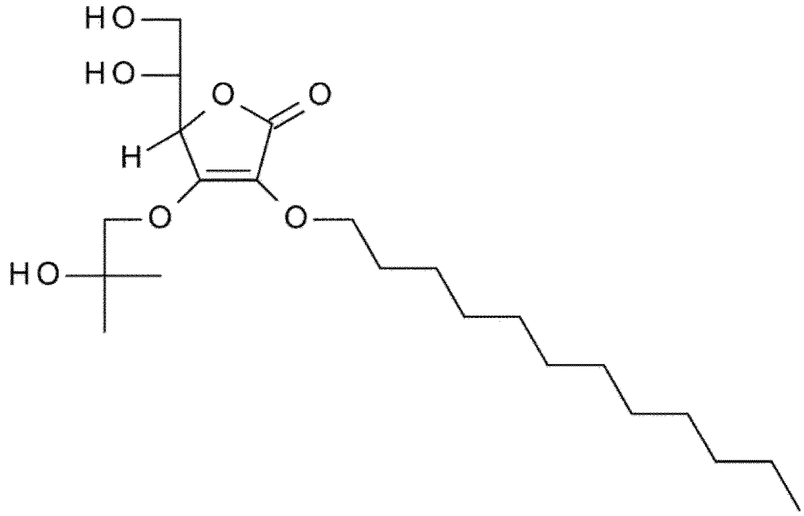

Synthesis Example 24: Synthesis of 2-O-tetradecyl-3-O-(2-hydroxyisobutyl) ascorbic acid

According to the same manner as in Synthesis Example 18, except that sodium hydrogen carbonate (0.56 g) was used instead of triethylamine and tetradecyl bromide (2.01 g) was used instead of diethyl sulfate, 2-O-tetradecyl-3-O-(2-hydroxyisobutyl) ascorbic acid (383.9 mg) in the title was obtained.

[Chemical formula 25]

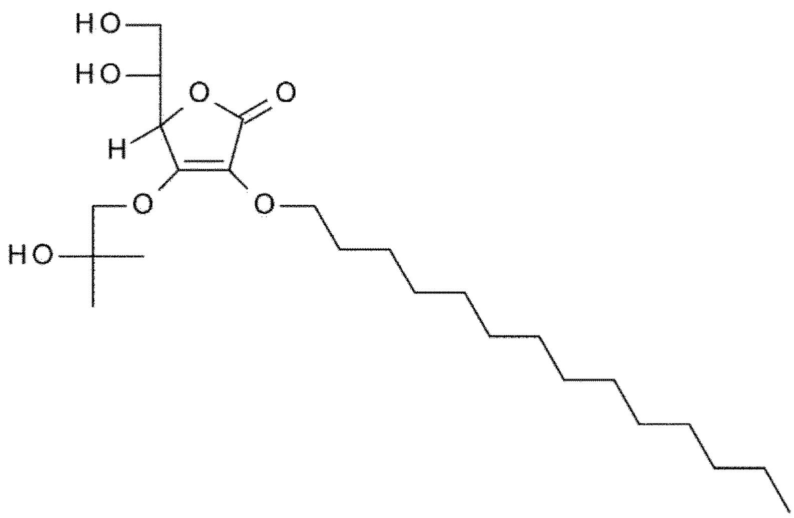

Synthesis Example 25: Synthesis of 2-O-hexadecyl-3-O-(2-hydroxyisobutyl) ascorbic acid

According to the same manner as in Synthesis Example 18, except that sodium hydrogen carbonate (0.56 g) was used instead of triethylamine and cetyl bromide (2.21 g) was used instead of diethyl sulfate, 2-O-hexadecyl-3-O-(2-hydroxyisobutyl) ascorbic acid (234.5 mg) in the title was obtained.

[Chemical formula 26]

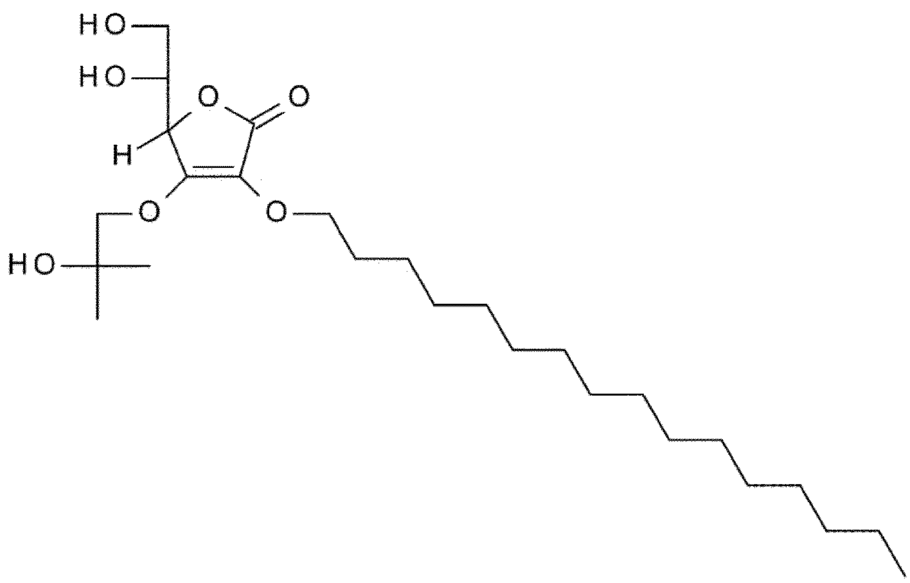

Synthesis Example 26: Synthesis of 2-O-allyl-3-O-(2-hydroxyisobutyl) ascorbic acid

According to the same manner as in Synthesis example 18, except that sodium hydrogen carbonate (0.70 g) was used instead of triethylamine and allyl bromide (0.88 g) was used instead of diethyl sulfate, 2-O-allyl-3-O-(2-hydroxyisobutyl) ascorbic acid (276.0 mg) in the title was obtained.

[Chemical formula 27]

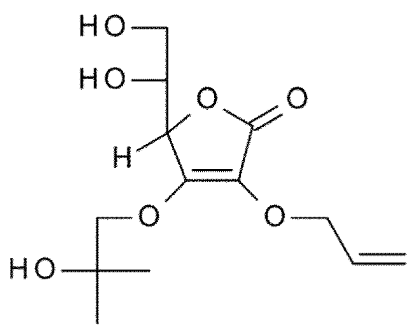

Synthesis Example 27 Synthesis of 2-O-benzyl-3-O-(2-hydroxyisobutyl) ascorbic acid

According to the same manner as in Synthesis Example 18, except that sodium hydrogen carbonate (1.12 g) was used instead of triethylamine and benzyl bromide (2.07 g) was used instead of diethyl sulfate, 2-O-benzyl-3-O-(2-hydroxyisobutyl) ascorbic acid (511.5 mg) in the title was obtained.

[Chemical formula 28]

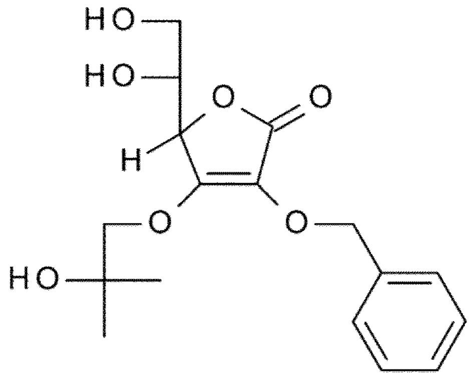

Synthesis Example 28: Synthesis of 2-O-glyceryl-3-O-(2-hydroxyisobutyl) ascorbic acid

According to the same manner as in Synthesis Example 13, except that 2-O-glyceryl ascorbic acid (3.0 g) was used instead of 3-O-glyceryl ascorbic acid, 2 -O-glyceryl-3-O-(2-hydroxyisobutyl) ascorbic acid (243.7 mg) in the title was obtained.

[Chemical formula 29]

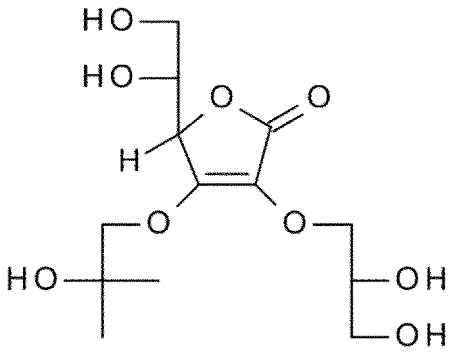

Synthesis Example 29: Synthesis of 2-O-(2-hydroxy-3-O-butyl)-3-O-(2-hydroxyisobutyl) ascorbic acid

According to the same manner as in Synthesis example 18, except that sodium hydrogen carbonate (0.20 g) was used instead of triethylamine and butyl glycidyl ether (1.89 g) was used instead of diethyl sulfate, 2-O-(2-hydroxy-3-O-butyl)-3-O-(2-hydroxyisobutyl) ascorbic acid (579.4 mg) was obtained.

[Chemical formula 30]

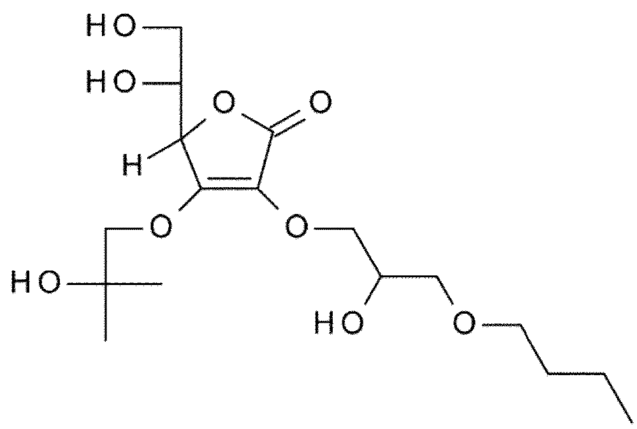

Synthesis Example 30: Synthesis of 2-O-(2-hydroxy-3-O-ethylhexyl)-3-O-(2-hydroxyisobutyl) ascorbic acid

According to the same manner as in Synthesis example 18, except that sodium hydrogen carbonate (0.20 g) was used instead of triethylamine and ethylhexyl glycidyl ether (2.70 g) was used instead of diethyl sulfate, 2-O-(2-hydroxy-3-O-ethylhexyl)-3-O-(2-hydroxyisobutyl) ascorbic acid (358.8 mg) was obtained.

[Chemical formula 31]

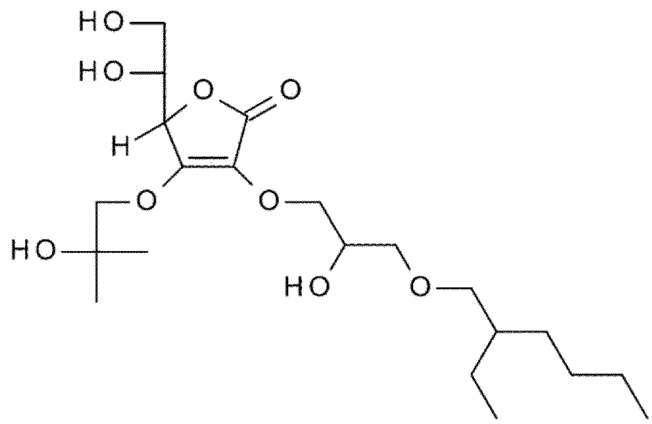

Synthesis Example 31 Synthesis of 2-O-(2-hydroxy-3-O-dodecyl)-3-O-(2-hydroxyisobutyl) ascorbic acid

According to the same manner as in Synthesis example 18, except that sodium hydrogen carbonate (0.10 g) was used instead of triethylamine and lauryl glycidyl ether (1.76 g) was used instead of diethyl sulfate, 2-O-(2-hydroxy-3-O-dodecyl)-3-O-(2-hydroxyisobutyl) ascorbic acid (458.0 mg) in the title was obtained.

[Chemical formula 32]

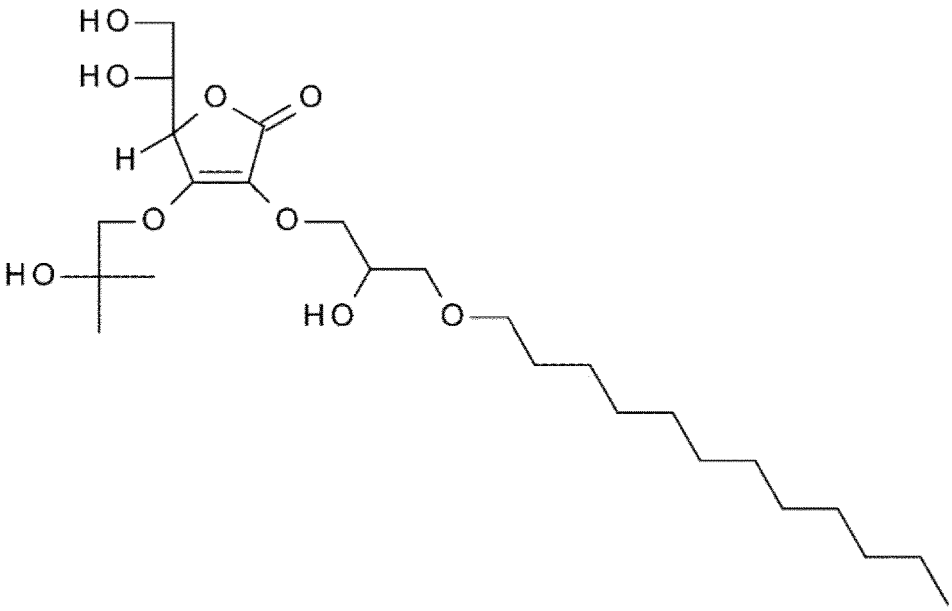

Synthesis Example 32 Synthesis of 2-O-(2-hydroxyisobutyl)-3-O-propylascorbic acid

According to the same manner as in Synthesis Example 3, except that sodium hydrogen carbonate (1.27 g) was used instead of triethylamine and propyl bromide (0.97 g) was used instead of diethyl sulfate, 2-O-(2-hydroxyisobutyl)-3-O-butylascorbic acid (642.7 mg) in the title was obtained.

[Chemical formula 33]

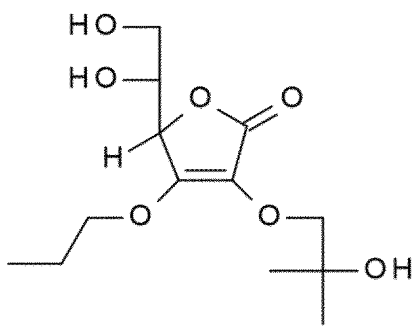

Synthesis Example 33: Synthesis of 2-O-propyl-3-O-(2-hydroxyisobutyl) ascorbic acid

According to the same manner as in Synthesis Example 18, except that sodium hydrogen carbonate (0.56 g) was used instead of triethylamine and propyl bromide (1.26 g) was used instead of diethyl sulfate, 2-O-propyl-3-O-(2-hydroxyisobutyl) ascorbic acid (423.7 mg) in the title was obtained.

[Chemical formula 34]

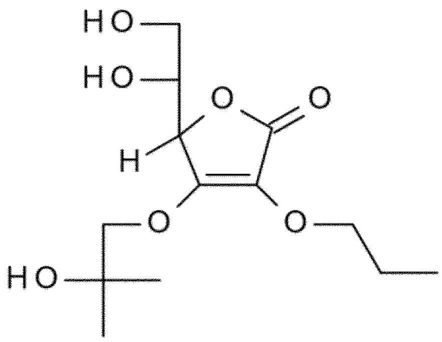

Synthesis Example 34: Synthesis of 2-O-(2-hydroxyisobutyl)-3-O-(2-hydroxypropyl) ascorbic acid

According to the same manner as in Synthesis Example 3, except that epoxypropane (0.64 g) was used instead of diethyl sulfate, 2-O-(2-hydroxyisobutyl)-3-O-(2-hydroxypropyl) ascorbic acid (0.17 g) in the title was obtained.

[Chemical formula 35]

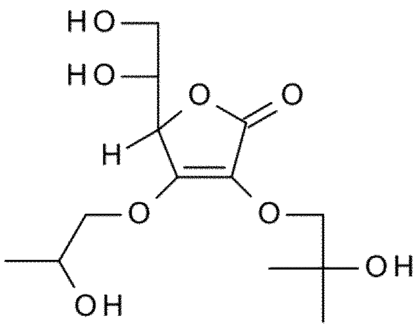

Synthesis Example 35: Synthesis of 2-O-(2-hydroxyisobutyl)-3-O-(2-hydroxybutyl) ascorbic acid

According to the same manner as in Synthesis Example 3, except that epoxybutane (0.80 g) was used instead of diethyl sulfate, 2-O-(2-hydroxyisobutyl)-3-O-(2-hydroxybutyl) ascorbic acid (0.18 g) in the title was obtained.

[Chemical formula 36]

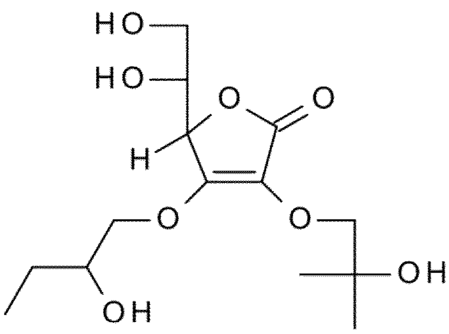

Synthesis Example 36: Synthesis of 2-O-(2-hydroxyisobutyl)-3-O-(2-hydroxydecyl) ascorbic acid

According to the same manner as in Synthesis example 3, except that sodium hydrogen carbonate (0.14 g) and tetrabutylammonium bromide (0.77 g) were used instead of triethylamine, and epoxydecane (1.63 g) was used instead of diethyl sulfate, 2-O-(2-hydroxyisobutyl)-3-O-(2-hydroxydecyl) ascorbic acid (0.11 g) was obtained.

[Chemical formula 37]

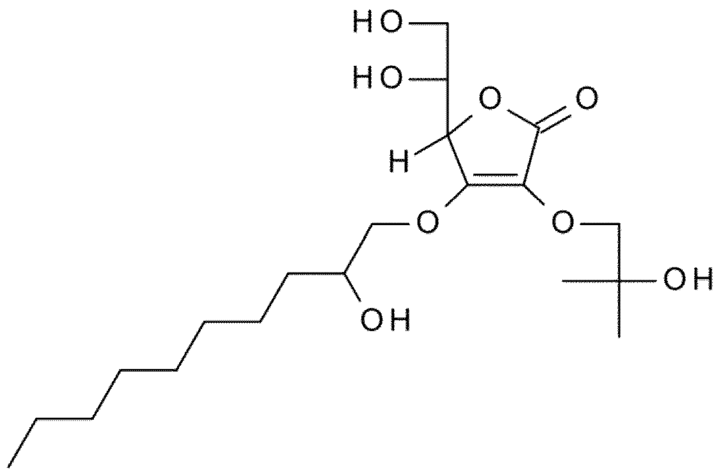

Synthesis Example 37: Synthesis of 2-O-(2-hydroxyisobutyl)-3-O-(2-hydroxyhexadecyl) ascorbic acid

According to the same manner as in Synthesis Example 3, except that epoxyhexadecane (2.65 g) was used instead of diethyl sulfate, 2-O-(2-hydroxyisobutyl)-3-O-(2-hydroxyhexadecyl) ascorbic acid (0.55 g) in the title was obtained.

[Chemical formula 38]

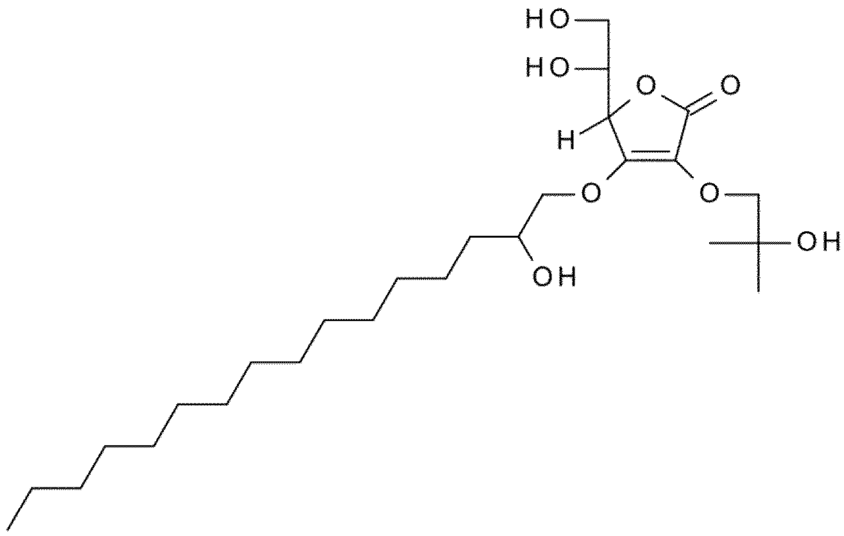

Synthesis Example 38: Synthesis of 2-O-(2-hydroxyisobutyl)-3-O-(2-hydroxyethylbenzene) ascorbic acid

According to the same manner as in Synthesis Example 3, except that styrene oxide (1.32 g) was used instead of diethyl sulfate, 2-O-(2-hydroxyisobutyl)-3-O-(2-hydroxyethylbenzene) ascorbic acid (0.65 g) in the title was obtained.

[Chemical formula 39]

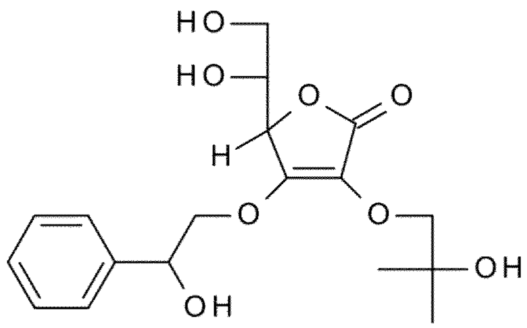

Synthesis Example 39: Synthesis of 3-O-(2-hydroxyisobutyl)-2-O-(2-hydroxypropyl) ascorbic acid

According to the same manner as in Synthesis example 18, except that diazabicycloundecene (0.15 g) was used instead of DMF, DMAc (5.0 mL) was used instead of triethylamine, and epoxypropane (0.35 g) was used instead of diethyl sulfate, 3-O-(2-hydroxyisobutyl)-2-O-(2-hydroxypropyl) ascorbic acid (0.38 g) in the title was obtained.

[Chemical formula 40]

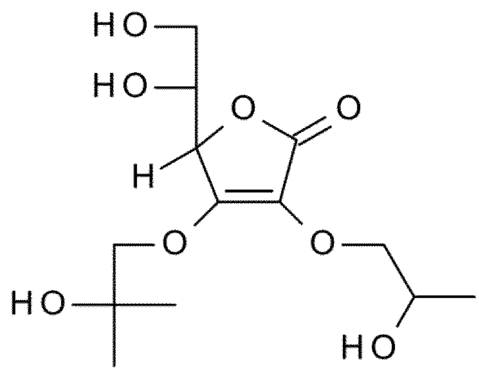

Synthesis Example 40: Synthesis of 3-O-(2-hydroxyisobutyl)-2-O-(2-hydroxybutyl) ascorbic acid

According to the same manner as in Synthesis example 18, except that diazabicycloundecene (0.15 g) was used instead of DMF, DMAc (5.0 mL ) was used instead of triethylamine, and epoxybutane (0.43 g) was used instead of diethyl sulfate, 3-O-(2-hydroxyisobutyl)-2-O-(2-hydroxybutyl)ascorbic acid (0.27 g) in the title was obtained.

[Chemical formula 41]

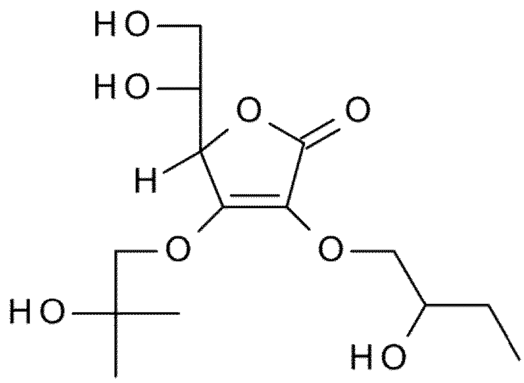

Synthesis Example 41 Synthesis of 3-O-(2-hydroxyisobutyl)-2-O-(2-hydroxydecyl) ascorbic acid

According to the same manner as in Synthesis example 18, except that diazabicycloundecene (0.15 g) was used instead of DMF, DMAc (5.0 mL) was used instead of triethylamine, and epoxydecane (0.94 g) was used instead of diethyl sulfate, 3-O-(2-hydroxyisobutyl)-2-O-(2-hydroxydecyl) ascorbic acid (0.30 g) in the title was obtained.

[Chemical formula 42]

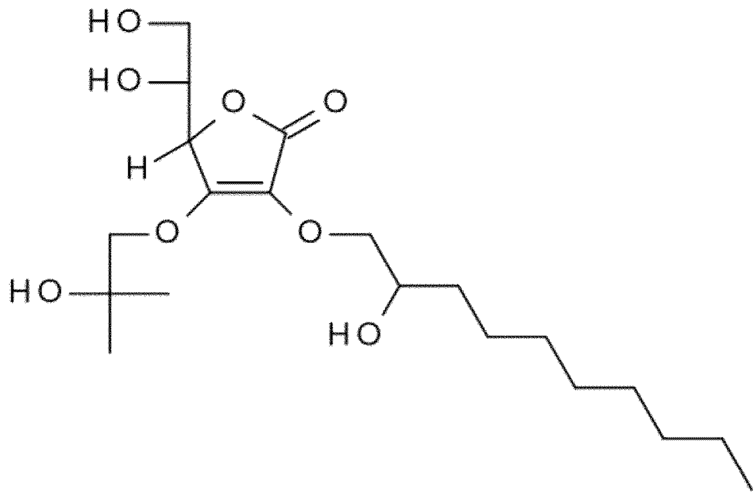

Synthesis Example 42: Synthesis of 3-O-(2-hydroxyisobutyl)-2-O-(2-hydroxyhexadecyl) ascorbic acid

According to the same manner as in Synthesis example 18, except that diazabicycloundecene (0.15 g) was used instead of DMF, DMAc (5.0 mL) was used instead of triethylamine, and epoxyhexadecane (1.44 g) was used instead of diethyl sulfate, 3-O-(2-hydroxyisobutyl)-2-O-(2-hydroxyhexadecyl) ascorbic acid (0.30 g) was obtained.

[Chemical formula 43]

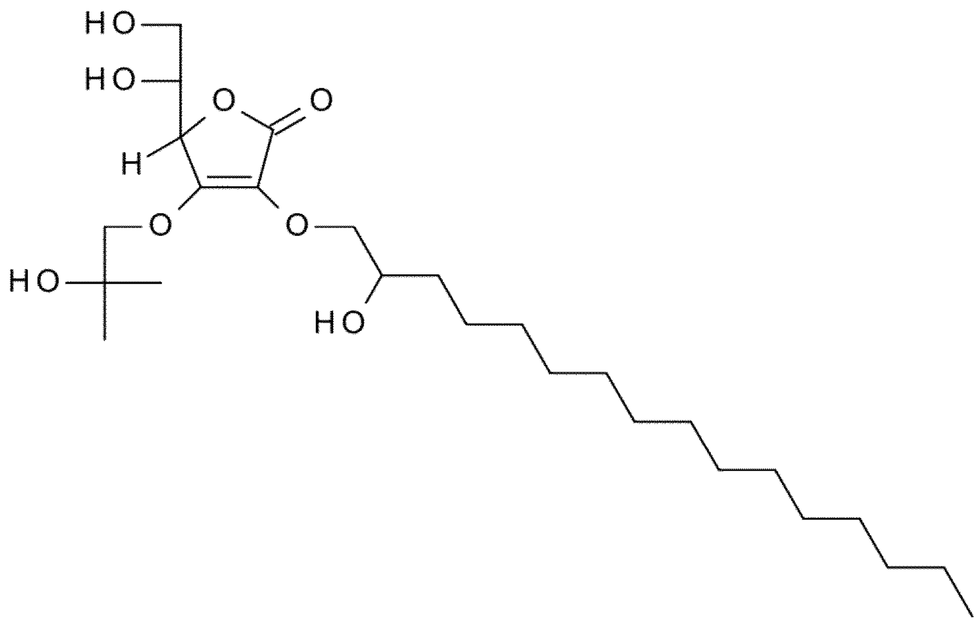

Synthesis Example 43: Synthesis of 3-O-(2-hydroxyisobutyl)-2-O-(2-hydroxyethylbenzene) ascorbic acid

According to the same manner as in Synthesis example 18, except that diazabicycloundecene (0.15 g) was used instead of DMF, DMAc (5.0 mL) was used instead of triethylamine, and styrene oxide (0.74 g) was used instead of diethyl sulfate, 3-O-(2-hydroxyisobutyl)-2-O-(2-hydroxyethylbenzene) ascorbic acid (0.48 g) in the title was obtained.

[Chemical formula 44]

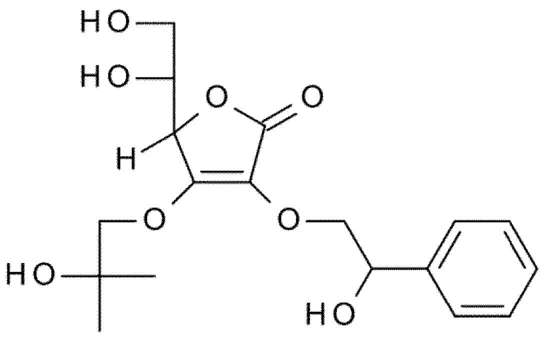

Mass spectrometry of the reaction products obtained in Synthesis Examples 1 to 43 was carried out using an LCMS-2020 (manufactured by Shimadzu Corporation). The measurement results are shown in Table 1.

[Table 1]

| Synthesis Example Number | Ionization | Actual measurements |
|---|---|---|
| 1 | [M-H] - | 247 |
| 2 | [M-H] - | 247 |
| 3 | [M+HCOO] - | 321 |
| 4 | [M+HCOO] - | 349 |
| 5 | [M+HCOO] - | 377 |
| 6 | [M+HCOO] - | 405 |
| 7 | [M+HCOO] - | 433 |
| 8 | [M+HCOO] - | 461 |
| 9 | [M+HCOO] - | 489 |
| 10 | [M+HCOO] - | 517 |
| 11 | [M+HCOO] - | 333 |
| 12 | [M+HCOO] - | 383 |
| 13 | [M+HCOO] - | 367 |
| 14 | [M+HCOO] - | 423 |
| 15 | [M+HCOO] - | 479 |
| 16 | [M+HCOO] - | 535 |
| 17 | [M+HCOO] - | 365 |
| 18 | [M+HCOO] - | 321 |
| 19 | [M+HCOO] - | 349 |
| 20 | [M+HCOO] - | 377 |
| 21 | [M+HCOO] - | 405 |
| 22 | [M+HCOO] - | 433 |
| 23 | [M+HCOO] - | 461 |
| 24 | [M+HCOO] - | 489 |
| 25 | [M+HCOO] - | 517 |
| 26 | [M+HCOO] - | 333 |
| 27 | [M+HCOO] - | 383 |

(continued)

| Synthesis Example Number | Ionization | Actual measurements |
|---|---|---|
| 28 | $[M+HCOO]^-$ | 367 |
| 29 | $[M+HCOO]^-$ | 423 |
| 30 | $[M+HCOO]^-$ | 479 |
| 31 | $[M+HCOO]^-$ | 535 |
| 32 | $[M+HCOO]^-$ | 335 |
| 33 | $[M+HCOO]^-$ | 335 |
| 34 | $[M+H]^+$ | 307 |
| 35 | $[M+H]^+$ | 321 |
| 36 | $[M+H]^+$ | 405 |
| 37 | $[M+H]^+$ | 489 |
| 38 | $[M+H]^+$ | 369 |
| 39 | $[M+H]^+$ | 307 |
| 40 | $[M+H]^+$ | 321 |
| 41 | $[M+H]^+$ | 405 |
| 42 | $[M+H]^+$ | 489 |
| 43 | $[M+H]^+$ | 369 |
| *Ionization was performed by electrospray ionization (ESI). | | |

The reaction products obtained in Synthesis Examples 1 to 43 were subjected to $^1$H-NMR using a JNM-ECS400 (manufactured by JEOL Ltd.). The measurement results are shown in Tables 2 to 5.

[Table 2]

| Synthesis Example Number | Measurement conditions | Chemical shift δ (ppm) |
|---|---|---|
| 1 | 400MHz, $CD_3OD$ | 1.24(6H, s), 3.68(2H, m), 3.76(2H, dd), 3.96(1H, td), 4.53(1H. d) |
| 2 | 400MHz, $CD_3OD$ | 1.25(3H, s), 1.26(3H, s), 3.66(2H, d), 3.91(1H, td), 4.34(2H, q), 4.83(1H, d) |
| 3 | 400MHz, $CD_3OD$ | 1.25(6H, s), 1.38(3H, s), 3.64(2H, dd), 3.83(2H, m), 3.83(1H, m), 4.57(2H, m), 4.82(1H, d) |
| 4 | 400MHz, $CD_3OD$ | 0.97(3H, t), 1.25(6H, s), 1.46(2H, sextet), 1.74(2H, quintet), 3.64(2H, d), 3.82(2H, m), 3.83(1H, m), 4.53(2H, m), 4.83(1H, d) |
| 5 | 400MHz, $CD_3OD$ | 0.92(3H, t), 1.24(6H, s), 1.34(2H, m), 1.34(2H, m), 1.44(2H, quintet), 1.76(2H, quintet), 3.64(2H, d), 3.82(2H, m), 3.83(1H, m), 4.52(2H, m), 4.83(1H, d) |
| 6 | 400MHz, $CD_3OD$ | 0.90(3H, t), 1.24(6H, s), 1.30-1.34(8H, br ), 3.64(2H, dd), 3.82(2H, m), 3.83(1H, m), 4.52(2H, m), 4.83(1H, d) |
| 7 | 400MHz, $CD_3OD$ | 0.89(3H, t), 1.25(6H, s), 1.29(12H, br ), 1.43(2H, m), 1.76(2H, m), 3.64(2H, d), 3.82(2H, m), 3.83(1H, m), 4.52(2H, m), 4.83(1H, br) |
| 8 | 400MHz, $CD_3OD$ | 0.89(3H, t), 1.25-1.36(16H, br ), 1.43(2H, m), 1.76(2H, m), 3.64(2H, dd), 3.82(2H, m), 3.83(1H, m), 4.52(2H, m), 4.83(1H, d) |

(continued)

| Synthesis Example Number | Measurement conditions | Chemical shift δ (ppm) |
|---|---|---|
| 9 | 400MHz, $CD_3OD$ | 0.89(3H, t), 1.25-1.36(20H, br ), 1.43(2H, m), 1.76(2H, m), 3.64(2H, dd), 3.82(2H, m), 3.83(1H, m), 4.52(2H, m), 4.83(1H, d) |
| 10 | 400MHz, $CD_3OD$ | 0.90(3H, t), 1.25(6H, s), 1.29(24H, br ), 1.44(2H, m), 1.76(2H, m), 3.64(2H, dd), 3.83(2H, m), 3.83(1H, m), 4.53(2H, m), 4.83(1H, d) |
| 11 | 400MHz, $CD_3OD$ | 1.25(6H, s), 3.65(2H, dd), 3.82(2H, m), 3.85(1H, m), 4.87(1H, d), 5.04(2H, m), 5.29(1H, dd), 5.43(1H, dd), 6.05(1H, m) |
| 12 | 400MHz, $CD_3OD$ | 1.25(6H, s), 3.65(2H, dd), 3.84(2H, q), 3.89(1H, td), 5.58(2H, q), 7.26(1H, m), 7.38(2H, m), 7.45(2H, d) |

[Table 3]

| Synthesis Example Number | Measurement conditions | Chemical shift δ (ppm) |
|---|---|---|
| 13 | 400MHz, $CD_3OD$ | 1.26(6H, s), 3.60(2H, d), 3.65(2H, d), 3.85(2H, m), 3.91(1H, m), 3.91(1H, m), 4.52(1H, m), 4.62(1H, m) |
| 14 | 400MHz, $CD_3OD$ | 0.93(3H, s), 1.26(6H, s), 1.38(2H, sextet), 1.56(2H, quintet), 3.48(2H, m), 3.50(2H, m), 3.65(2H, dd), 3.85(2H, m), 3.90(1H, m), 4.03(1 H, m), 4.52(1H, m), 4.62(1H, m), 4.87(1H, d) |
| 15 | 400MHz, $CD_3OD$ | 0.88(3H, s), 0.88(3H, t), 1.26(6H, s), 1.14-1.41(9H, m), 3.49(2H, m), 3.53(2H, m), 3.65(2H, dd), 3.85(2H, m), 3.90(1H, m), 4.03( 1H, m), 4.51(1H, m), 4.60(1H, m) |
| 16 | 400MHz, $CD_3OD$ | 0.90(3H, t), 1.26(6H, s), 1.26-1.32(18H, br), 3.47(2H, t), 3.51(2H, dd), 3.65(2H, d), 3.84(1H, d) , 3.87(1H, d), 3.90(1H, m), 4.04(1H, m), 4.52(1H, m), 4.63(1H, m), 4.88(1H, m) |
| 17 | 400MHz, $CD_3OD$ | 1.26(6H, s), 1.26(6H, s), 3.66(2H, d), 3.85(2H, m), 3.93(1H, td), 4.38(2H, m), 4.89(1H, d) |
| 18 | 400MHz, $CD_3OD$ | 1.26(6H, s), 1.30(3H, t), 3.66(2H, d), 3.92(1H, td), 4.07(2H, qd ), 4.32(2H, m) |
| 19 | 400MHz, $CD_3OD$ | 0.96(3H, t), 1.26(6H, s), 1.46(2H, sextet), 1.68(2H, quintet), 3.66(2H, d), 3.92(1H, td), 4.02(2H, td), 4.32 (2H, m) |
| 20 | 400MHz, $CD_3OD$ | 0.91(3H, t), 1.26(2H, s), 1.33(2H, m), 1.33(2H, m), 1.43(2H, quintet), 1.69(2H, sextet), 3.66(2H, d), 3.92 (1H, td), 4.01(2H, td), 4.32(2H, m) |
| 21 | 400MHz, $CD_3OD$ | 0.90(3H, t), 1.26(6H, s), 1.31-1.49(10H, br ), 1.69(2H, quintet), 3.66(2H, d), 3.92(1H, td), 4.01(2H, td) , 4.33(2H, q), 4.89(1H, d) |

[Table 4]

| Synthesis Example Number | Measurement conditions | Chemical shift δ (ppm) |
|---|---|---|
| 22 | 400MHz, $CD_3OD$ | 0.89(3H, 1.29-1.36(12H, br), 1.43(2H, m), 1.69(2H, quintet), 3.66(2H, d), 3.92(1H, td), 4.01(2H, td), 4.32(2H, m) |
| 23 | 400MHz, $CD_3OD$ | 0.89(3H, t), 1.24-1.37(16H, br), 1.43(2H, m), 1.69(2H, quintet), 3.66(2H, d), 3.92(1H, td), 4.01(2H, t), 4.32(2H, m) |

(continued)

| Synthesis Example Number | Measurement conditions | Chemical shift δ (ppm) |
|---|---|---|
| 24 | 400MHz, $CD_3OD$ | 0.89(3H, t), 1.24-1.37(20H, br), 1.43(2H, m), 1.69(2H, quintet), 3.66(2H, d), 3.92(1H, td), 4.01(2H, t), 4.32(2H, m) |
| 25 | 400MHz, $CD_3OD$ | 0.89(3H, t), 1.24-1.37(24H, br), 1.43(2H, m), 1.69(2H, quintet), 3.66(2H, d), 3.92(1H, td), 4.01(2H, t), 4.32(2H, m) |
| 26 | 400MHz, $CD_3OD$ | 1.25(6H, s), 3.66(2H, d), 3.92(1H, td), 4.32(2H, m), 4.54(2H, dd), 4.89(1H, d), 5.26(1H, dd), 5.35(1H, dd), 6.04(1H, tdd) |
| 27 | 400MHz, $CD_3OD$ | 1.13(6H, s), 3.66(2H, d), 3.89(1H, td), 4.01(2H, s), 5.04(2H, m), 7.33-7.43(5H, m) |
| 28 | 400MHz, $CD_3OD$ | 1.26(6H, s), 3.59(2H, d), 3.66(2H, d), 3.86(1H, m), 3.97(2H, m), 4.16(1H, td), 4.40(2H, m), 4.90(1H, d) |
| 29 | 400MHz, $CD_3OD$ | 0.92(3H, t), 1.26(6H, s), 1.38(2H, sextet), 1.55(2H, quintet), 3.47(2H, m), 3.47(2H, m), 3.66(2H, d), 3.92(1H, m), 3.98(2H, m), 4.15(1 H, quintet), 4.39(2H, m), 4.89(1H, br) |
| 30 | 400MHz, $CD_3OD$ | 0.88(3H, t), 0.88(3H, t), 1.26-1.42(8H, m), 1.50(1H, m), 3.35(2H, m), 3.46(2H, m), 3.66(2H, d), 3.98(2H, m), 4.17(1H, quintet), 4.3 9(2H, m) |
| 31 | 400MHz, $CD_3OD$ | 0.89(3H, t), 1.26(6H, s), 1.28-1.36(18H, br), 1.57(2H, quintet), 3.46(2H, m), 3.46(2H, m), 3.66(2H, d) , 3.97(2H, m), 4.16(1H, quintet), 4.40(2H, m) |
| 32 | 400MHz, $CD_3OD$ | 1.17 (3H, t), 1.2 0 (2 H, m), 1.2 5 (6H, s), 3.64 (2H, d), 3. 84 (2H, m), 3.84 (1H, m), 4.4 9 (2H, td) , 4. 84 ( 1H , br) |
| 33 | 400MHz, $CD_3OD$ | 1.00 (3H, t), 1. 71 ( 2 H, m), 1.2 6 (6H, s), 3.6 6 (2H, d d), 3. 92 (1 H, m), 3.98 (2 H, td), 4. 33 (2H, m), 4. 89 (1H, d) |

[Table 5]

| Synthesis Example Number | Measurement conditions | Chemical shift δ (ppm) |
|---|---|---|
| 34 | 400MHz, $CD_3OD$ | 1.19(3H, m), 1.25(6H, s), 3.62(2H, dd), 3.83(2H, d), 3.87(1H, m), 4.06(1H, sextet), 4.38(1H, m), 4.48(1H, m), 4.87(1H, br ) |
| 35 | 400MHz, $CD_3OD$ | 1.00(3H, t), 1.25(6H, s), 1.53(2H, m), 3.66(2H, d), 3.83(4H, m), 4.41(1H, m), 4.52(1H, m), 4.87(1H, br ) |
| 36 | 400MHz, $CD_3OD$ | 0.90(3H, t), 1.25(6H, s), 1.31(12H, br ), 1.50(2H, m), 3.66(2H, d), 3.87(4H, m), 4.40(1H, m), 4.52 (1H, m), 4.85(1H, m) |
| 37 | 400MHz, $CD_3OD$ | 0.90(3H, t), 1.26(6H, s), 1.28(24H, br ), 1.50(2H, m), 3.66(2H, d), 3.89(4H, m), 4.40(1H, m), 4.52 (1H, m), 4.87(1H, br ) |
| 38 | 400MHz, $CD_3OD$ | 1.23(6H, s), 3.48(1H, d), 3.69(2H, dd), 3.80(1H, d), 3.83(2H, m), 4.01(1H, td), 7.33-7.43(5H, m) |
| 39 | 400MHz, $CD_3OD$ | 1.18 ( 3H, d), 1.26(6H, s), 3.65(2H, d), 3.82(1H, m), 3.93(1H, m), 4.02(2H, m), 4.39(2H, m) |
| 40 | 400MHz, $CD_3OD$ | 0.98(3H, t), 1.26 (6H, s), 1.51 (2H, m), 3.65(2H, d), 3.86(1H, m), 3.93(1H, m), 4.06(2H, m), 4.39(2H, m) |

(continued)

| Synthesis Example Number | Measurement conditions | Chemical shift δ (ppm) |
|---|---|---|
| 41 | 400MHz, $CD_3OD$ | 0.89(3H, t), 1.26(6H, s), 1.30(12H, br ), 1.48(2H, m), 3.65(2H, d), 3.86(1H, m), 3.93(1H, m), 4.05 (2H, m), 4.40(2H, m) |
| 42 | 400MHz, $CD_3OD$ | 0.89(3H, t), 1.26(6H, s), 1.28(24H, br ), 1.48(2H, m), 3.65(2H, d), 3.86(1H, m), 3.93(1H, m), 4.06 (2H, m), 4.40(2H, m) |
| 43 | 400MHz, $CD_3OD$ | 1.19(6H, s), 3.64(2H, d), 3.91(1H, td), 4.07(1H, m), 4.21(1H, m), 4.27(2H, s), 4.87(1H, m) , 4.94(1H, dd), 7.25(1H, t), 7.32(2H, t), 7.39(2H, d) |

The products obtained in Synthesis Examples 1 to 43 were subjected to $^{13}$C-NMR using a JNM-ECS400 (manufactured by JEOL Ltd.). The measurement results are shown in Tables 6 to 8.

[Table 6]

| Synthesis Example Number | Measurement conditions | Chemical shift δ (ppm) |
|---|---|---|
| 1 | 100MHz, $CD_3OD$ | 26.1, 62.7, 71.3, 71.8, 78.5, 81.4, 119.8, 175.9, 176.1 |
| 2 | 100MHz, $CD_3OD$ | 25.9, 63.3, 70.5, 70.8, 76.7, 79.5, 120.8, 152.1, 173.0 |
| 3 | 100MHz, $CD_3OD$ | 15.5, 26.3, 63.2, 69.6, 70.5, 70.8, 76.6, 81.5, 122.6, 159.4, 172.3 |
| 4 | 100MHz, $CD_3OD$ | 14.1, 19.9, 26.3, 32.7, 63.3, 70.6, 70.8, 73.5, 76.5, 81.6, 122.7, 159.6, 172.3 |
| 5 | 100MHz, $CD_3OD$ | 14.4, 23.6, 26.4, 26.4, 30.6, 32.7, 63.3, 70.6, 70.8, 73.7, 76.5, 81.6, 122.7, 159.6, 172.3 |
| 6 | 100MHz, $CD_3OD$ | 14.5, 23.7, 26.3, 26.7, 30.4, 30.4, 30.6, 33.0, 63.2, 70.6, 70.8, 73.7, 76.5, 81.5, 122.7, 159.6, 172.3 |
| 7 | 100MHz, $CD_3OD$ | 14.4, 23.7, 26.4, 26.7, 30.4-30.7, 33.1, 63.3, 70.6, 70.8, 73.7, 76.5, 81.6, 122.7, 159.6, 172.3 |
| 8 | 100MHz, $CD_3OD$ | 14.5, 23.7, 26.4, 26.7, 30.4-30.8, 33.1, 63.3, 70.6, 70.8, 73.7, 76.5, 81.6, 122.7, 159.6, 172.3 |
| 9 | 100MHz, $CD_3OD$ | 14.5, 23.7, 26.4, 26.7, 30.4-30.8, 33.1, 63.3, 70.6, 70.8, 73.7, 76.5, 81.6, 122.7, 159.6, 172.3 |
| 10 | 100MHz, $CD_3OD$ | 14.5, 23.8, 26.3, 26.7-33.1, 63.2, 70.6, 70.8, 73.7, 76.5, 81.5, 122.7, 159.6, 172.3 |
| 11 | 100MHz, $CD_3OD$ | 26.3, 63.3, 70.5, 70.8, 73.7, 76.6, 81.4, 118.7, 123.0, 133.7, 158.9, 172.1 |
| 12 | 100MHz, $CD_3OD$ | 26.3, 63.3, 70.5, 70.8, 74.9, 76.6, 81.5, 123.3, 129.0, 129.6, 129.6, 137.3, 159.1, 172.1 |
| 13 | 100MHz, $CD_3OD$ | 26.3, 63.2, 63.5, 70.5, 70.8, 71.6, 74.3, 76.7, 81.4, 123.1, 159.2, 172.0 |
| 14 | 100MHz, $CD_3OD$ | 14.3, 20.3, 26.4, 32.8, 63.2, 70.1, 70.1, 70.5, 70.8, 72.5, 74.7, 76.7, 81.4, 123.1, 159.2, 172.0 |
| 15 | 100MHz, $CD_3OD$ | 11.4, 14.5, 24.2, 24.9, 26.4, 30.2, 31.6, 40.9, 63.2, 70.2, 70.2, 70.5, 70.8, 72.7, 74.7, 76.7, 81.4, 123.1, 159.2, 174.8 |

(continued)

| Synthesis Example Number | Measurement conditions | Chemical shift δ (ppm) |
|---|---|---|
| 16 | 100MHz, $CD_3OD$ | 14.5, 23.8, 26.4, 27.2, 30.5, 30.8, 30.8(br), 33.1, 63.2, 70.1, 70.5, 70.8, 72.5, 72.8, 74.7, 76.7, 81.4, 123.1, 159.2, 17 2.0 |
| 17 | 100MHz, $CD_3OD$ | 25.9, 26.4, 63.2, 70.6, 70.6, 70.8, 76.7, 80.4, 81.4, 123.0, 159.5, 172.1 |

[Table 7]

| Synthesis Example Number | Measurement conditions | Chemical shift δ (ppm) |
|---|---|---|
| 18 | 100MHz, $CD_3OD$ | 15.5, 26.0, 63.2, 69.5, 70.6, 70.6, 76.7, 80.1, 122.8, 159.8, 172.4 |
| 19 | 100MHz, $CD_3OD$ | 14.4, 20.1, 26.0, 32.9, 63.2, 70.6, 70.6, 73.6, 76.7, 80.1, 123.1, 159.5, 172.3 |
| 20 | 100MHz, $CD_3OD$ | 14.4, 23.7, 26.0, 26.8, 30.8, 32.7, 63.2, 70.6, 70.6, 73.9, 76.7, 80.2, 123.1, 159.5, 172.3 |
| 21 | 100MHz, $CD_3OD$ | 14.5, 23.7, 26.0, 27.1, 30.4, 30.5, 30.8, 33.0, 63.2, 70.6, 70.6, 73.9, 76.7, 80.2, 123.1, 159.5, 172.3 |
| 22 | 100MHz, $CD_3OD$ | 14.5, 23.7, 26.0, 27.1, 30.5, 30.8, 30.5-30.8, 63.2, 70.6, 70.6, 73.9, 76.7, 80.2, 123.1, 159.5, 172.3 |
| 23 | 100MHz, $CD_3OD$ | 14.5, 23.8, 26.0, 27.1, 30.5, 30.8, 30.8( br ), 33.1, 63.2, 70.6, 70.6, 73.9, 76.7, 80.2, 123.1, 159.5, 172.3 |
| 24 | 100MHz, $CD_3OD$ | 14.5, 23.8, 26.0, 27.1, 30.5, 30.8, 30.7-30.8, 33.1, 63.2, 70.6, 70.6, 73.9, 76.7, 80.2, 123.1, 159.5, 172.3 |
| 25 | 100MHz, $CD_3OD$ | 14.5, 23.8, 26.0, 27.1, 30.5, 30.8, 30.7-30.8, 33.1, 63.2, 70.6, 70.6, 73.9, 76.7, 80.2, 123.1, 159.5, 172.3 |
| 26 | 100MHz, $CD_3OD$ | 26.0, 63.2, 70.6, 70.6, 74.1, 76.7, 80.2, 119.2, 122.4, 134.5, 159.9, 172.2 |
| 27 | 100MHz, $CD_3OD$ | 25.8, 63.3, 70.4, 70.6, 75.3, 76.8, 80.2, 122.0, 129.7, 129.7, 130.2, 137.6, 160.3, 172.3 |
| 28 | 100MHz, $CD_3OD$ | 25.9, 63.2, 63.9, 70.6, 70.8, 71.9, 75.0, 76.8, 80.2, 122.8, 159.7, 172.4 |
| 29 | 100MHz, $CD_3OD$ | 14.3, 20.3, 26.0, 32.9, 63.2, 70.3, 70.6, 70.8, 72.3, 72.9, 75.3, 76.8, 80.3, 122.8, 159.6, 172.2 |
| 30 | 100MHz, $CD_3OD$ | 11.5, 14.5, 24.2, 24.9, 26.0, 30.3, 31.6, 41.0, 63.2, 70.3, 70.6, 70.8, 72.3, 72.9, 75.3, 76.8, 80.3, 122.8, 159.6, 172.2 |
| 31 | 100MHz, $CD_3OD$ | 14.5, 23.8, 26.0, 27.2, 30.5, 30.8, 30.6-30.8, 33.1, 63.2, 70.4, 70.6, 70.8, 72.7, 72.9, 76.8, 77.9, 80.3, 122.8, 159.6, 1 72.2 |
| 32 | 100MHz, $CD_3OD$ | 10.4, 23.9, 26.3, 63.3, 70.6, 70.8, 75.1, 76.5, 81.5, 122.7 159.6, 172.3 |
| 33 | 100MHz, $CD_3OD$ | 10.8, 24.0, 25.9, 63.2, 70.5, 70.6, 75.5, 76.6, 80.2, 123.1 159.5, 172.3 |

[Table 8]

| Synthesis Example Number | Measurement conditions | Chemical shift δ (ppm) |
|---|---|---|
| 34 | 100MHz, $CD_3OD$ | 19.13, 26.35, 26.36, 63.17, 70.49, 70.57, 70.81, 76.67, 76.69, 77.91, 77.94, 81.33, 122.98, 123.02, 159.26, 159.28, 172.09 |
| 35 | 100MHz, $CD_3OD$ | 10.25, 26.35, 26.37, 63.17, 70.50, 70.58, 70.81, 76.66, 76.69, 76.84, 81.31, 81.35, 122.99, 123.00, 159.35, 172.10, 172.12 |
| 36 | 100MHz, $CD_3OD$ | 14.47, 23.74, 26.30, 26.33, 30.44, 30.68, 30.75, 33.07, 63.15, 70.51, 70.57, 70.80, 76.65, 76.68, 77.16, 81.33, 81.35, 122 .98, 123.00, 159.33, 172.11 |
| 37 | 100MHz, $CD_3OD$ | 14.49, 23.76, 26.34, 26.37, 26.54, 26.58, 30.51, 30.77, 30.83, 33.10, 34.02, 63.15, 70.50, 70.58, 70.80, 76.65, 76.68, 77. 16, 81.33, 81.35, 122.97, 123.00, 159.33, 172.08, 172.11 |
| 38 | 100MHz, $CD_3OD$ | 2 6.24, 26.51, 63.12. 67.33, 70.50, 70.79, 76.97, 80.49, 85.24, 123.18, 127.54, 129.62, 138.32, 157.94, 171.83 |
| 39 | 100MHz, $CD_3OD$ | 19.46, 25.87, 25.91, 63.19, 67.08, 67.10, 70.57, 70.82, 70.84, 76.72, 76.74, 78.57, 78.70, 80.18, 80.22, 122.70, 122.77, 1 59.55, 159.61, 172.27, 172.31 |
| 40 | 100MHz, $CD_3OD$ | 10.27, 25.88, 25.91, 27.21, 27.31, 63.20, 70.58, 70.83, 70.86, 76.73, 76.75, 77.24, 77.36, 80.16, 80.21, 122.71, 122.79, 1 59.55, 159.61, 172.30, 172.34 |
| 41 | 100MHz, $CD_3OD$ | 14.47, 23.75, 25.89, 25.93, 26.63, 30.44, 30.71, 30.82, 33.08, 34.37, 63.19, 70.57, 70.83, 70.87, 76.73, 76.75, 77.24, 77. 36, 80.17, 80.22, 122.71, 122.78, 159.53, 159.60, 172.30, 172.34 |
| 42 | 100MHz, $CD_3OD$ | 14.47, 23.76, 25.89, 25.92, 26.63, 30.50, 30.81, 33.10, 34.36, 63.19, 70.57, 70.82, 70.86, 76.72, 76.74, 77.56, 77.71, 80. 16, 80.22, 122.70, 122.78, 159.51, 159.57, 172.28, 172.33 |
| 43 | 100MHz, $CD_3OD$ | 2 5.88, 25.90, 63.16, 70.54, 70.80, 73.39, 76.76, 77.89, 80.15, 122.41, 127.48, 129.41, 142.28, 159.55, 172.24 |

Test Example 1 [Melanin production inhibitory effect]

As a test of the whitening effect, the effect on theophylline-induced melanin production in B16 melanoma 4A5 cells was evaluated for the ascorbic acid derivatives of the present invention according to the following procedure. A similar evaluation was also performed for a known ascorbic acid derivative for comparison. The results are shown in Tables 9 to 11.

(1) B16 mouse melanoma 4A5 cells were seeded in a 48-well plate at a cell density of $8.0\times10^3$ cells/well.
(2) After culturing for 24 hours in Dulbecco's modified Eagle's medium (manufactured by SIGMA, hereinafter abbreviated as "D-MEM") containing 10% fetal bovine serum, the medium was replaced with D-MEM containing 10% fetal bovine serum containing theophylline (final concentration 1 mmol/L) and a specified concentration of a sample.
(3) After culturing for 3 days in the presence of the sample, the medium was removed using an aspirator, distilled water was added, and the cells were disrupted by ultrasonication.
(4) The amount of protein was then quantified using a BCA protein assay kit (manufactured by PIERCE), and the amount of melanin produced was measured by an alkali solubilization method. Sodium hydroxide was added to the cell lysate to a final concentration of 1 mol/L, and the solution was dissolved by heating (60°C, 30 minutes), thereafter the absorbance at 405 nm was measured using a microplate reader. The amount of melanin was calculated from a calibration curve prepared using synthetic melanin (SIGMA) as a standard. The amount of melanin per unit protein was calculated by dividing the amount of melanin by the amount of protein.
(5) The melanin production inhibition rate was calculated using the following formula.

$$\text{Melanin production inhibition rate (\%)} = [1 - (A - B) / (C - B)] \times 100$$

[In the formula, A represents the amount of melanin per unit protein (g/g) when the sample was added, B represents the amount of melanin per unit protein (g/g) in the normal group, and C represents the amount of melanin per unit protein (g/g) in the control group.]

The melanin production inhibition rate when the sample was measured at a concentration of 10 mM or less is expressed as follows. The measurement was performed with N=4.

| | | |
|---|---|---|
| <20% | : | + |
| 20-40% | : | + |
| 40-60% | : | ++ |
| 60-100% | : | +++ |

[Table 9]

| Example number | Ascorbic acid derivatives or salts thereof | Melanin production inhibition effect |
|---|---|---|
| Comparison | 2-O-glyceryl ascorbic acid | + |
| Comparison | 3-O-glyceryl ascorbic acid | + |
| 1 | 2-O-(2-hydroxyisobutyl)ascorbic acid (Synthesis Example 1) | ++ |
| 2 | 3-O-(2-hydroxyisobutyl)ascorbic acid (Synthesis Example 2) | + |
| 3 | 2-O-(2-hydmxyisobutyl)-3-O-ethylascorbic acid (Synthesis Example 3) | +++ |
| 4 | 2-O-(2-hydroxyisobutyl)-3-O-hexyl ascorbic acid (Synthesis Example 5) | + |
| 5 | 2-O-(2-hydmxyisobutyl)-3-O-octyl ascorbic acid (Synthesis Example 6) | +++ |
| 6 | 2-O-(2-hydmxyisobutyl)-3-O-decyl ascorbic acid (Synthesis Example 7) | +++ |
| 7 | 2-O-(2-hydroxyisobutyl)-3-O-dodecyl ascorbic acid (Synthesis Example 8) | ++ |
| 8 | 2-O-(2-hydroxyisobutyl)-3-O-tetradecyl ascorbic acid (Synthesis Example 9) | ++ |
| 9 | 2-O-(2-hydroxyisobutyl)-3-O-hexadecylascorbic acid (Synthesis Example 10) | + |
| 10 | 2-O-(2-hydroxyisobutyl)-3-O-allylascorbic acid (Synthesis Example 11) | +++ |
| 11 | 2-O-(2-hydroxyisobutyl)-3-O-benzyl ascorbic acid (Synthesis Example 12) | +++ |
| 12 | 2-O-(2-hydroxyisobutyl)-3-O-glyceryl ascorbic acid (Synthesis Example 13) | ++ |
| 13 | 2-O-(2-hydroxyisobutyl)-3-O-(2-hydroxy-3-O-butyl) ascorbic acid (Synthesis Example 14) | ++ |
| 14 | 2-O-(2-hydroxyisobutyl)-3-O-(2-hydroxy-3-O-ethylhexyl) ascorbic acid (Synthesis Example 15) | + |
| 15 | 2-O-(2-hydroxyisobutyl)-3-O-(2-hydroxy-3-O-dodecyl) ascorbic acid (Synthesis Example 16) | + |

(continued)

| Example number | Ascorbic acid derivatives or salts thereof | Melanin production inhibition effect |
|---|---|---|
| 16 | 2,3-Di-O-(2-hydroxyisobutyl) ascorbic acid (Synthesis Example 17) | + |

[Table 10]

| Example Number | Ascorbic acid derivatives or salts thereof | Melanin production inhibition effect |
|---|---|---|
| 17 | 2-O-Ethyl-3-O-(2-hydroxyisobutyl) ascorbic acid (Synthesis Example 18) | +++ |
| 18 | 2-O-Butyl-3-O-(2-hydroxyisobutyl) ascorbic acid (Synthesis Example 19) | + |
| 19 | 2-O-Hexyl-3-O-(2-hydroxyisobutyl) ascorbic acid (Synthesis Example 20) | ++ |
| 20 | 2-O-Octyl-3-O-(2-hydroxyisobutyl) ascorbic acid (Synthesis Example 21) | ++ |
| 21 | 2-O-Decyl-3-O-(2-hydroxyisobutyl) ascorbic acid (Synthesis Example 22) | ++ |
| 22 | 2-O-dodecyl-3-O-(2-hydroxyisobutyl) ascorbic acid (Synthesis Example 23) | +++ |
| 23 | 2-O-Tetradecyl-3-O-(2-hydroxyisobutyl) ascorbic acid (Synthesis Example 24) | +++ |
| 24 | 2-O-Hexadecyl-3-O-(2-hydroxyisobutyl) ascorbic acid (Synthesis Example 25) | + |
| 25 | 2-O-Allyl-3-O-(2-hydroxyisobutyl) ascorbic acid (Synthesis Example 26) | + |
| 26 | 2-O-benzyl-3-O-(2-hydroxyisobutyl) ascorbic acid (Synthesis Example 27) | ++ |
| 27 | 2-O-Glyceryl-3-O-(2-hydroxyisobutyl) ascorbic acid (Synthesis Example 28) | + |
| 28 | 2-O-(2-hydroxy-3-O-ethylhexyl)-3-O-(2-hydroxyisobutyl) ascorbic acid (Synthesis Example 30) | + |
| 29 | 2-O-(2-hydroxy-3-O-dodecyl)-3-O-(2-hydroxyisobutyl) ascorbic acid (Synthesis Example 31) | + |
| 30 | 2-O-(2-hydroxyisobutyl)-3-O-propylascorbic acid (Synthesis Example 32) | ++ |
| 31 | 2-O-Propyl-3-O-(2-hydroxyisobutyl) ascorbic acid (Synthesis Example 33) | ++ |

[Table 11]

| Example Number | Ascorbic acid derivatives or salts thereof | Melanin production inhibition effect |
|---|---|---|
| 32 | 2-O-(2-hydroxyisobutyl)-3-O-(2-hydroxypropyl) ascorbic acid (Synthesis Example 34) | +++ |
| 33 | 2-O-(2-hydroxyisobutyl)-3-O-(2-hydroxybutyl) ascorbic acid (Synthesis Example 35) | ++ |
| 34 | 2-O-(2-hydroxyisobutyl)-3-O-(2-hydroxydecyl) ascorbic acid (Synthesis Example 36) | ++ |

(continued)

| Example Number | Ascorbic acid derivatives or salts thereof | Melanin production inhibition effect |
|---|---|---|
| 35 | 2-O-(2-hydroxyisobutyl)-3-O-(2-hydroxyhexadecyl) ascorbic acid (Synthesis Example 37) | + |
| 36 | 2-O-(2-hydroxyisobutyl)-3-O-(2-hydroxyethylbenzene) ascorbic acid (Synthesis Example 38) | ++ |
| 37 | 3-O-(2-hydroxyisobutyl)-2-O-(2-hydroxypropyl) ascorbic acid (Synthesis Example 39) | +++ |
| 38 | 3-O-(2-hydroxyisobutyl)-2-O-(2-hydroxybutyl) ascorbic acid (Synthesis Example 40) | ++ |
| 39 | 3-O-(2-hydroxyisobutyl)-2-O-(2-hydroxydecyl) ascorbic acid (Synthesis Example 41) | ++ |
| 40 | 3-O-(2-hydroxyisobutyl)-2-O-(2-hydroxyhexadecyl) ascorbic acid (Synthesis Example 42) | ++ |
| 41 | 3-O-(2-hydroxyisobutyl)-2-O-(2-hydroxyethylbenzene) ascorbic acid (Synthesis Example 43) | ++ |

The results in Tables 9 to 11 show that the ascorbic acid derivatives of the present invention have a whitening effect equal to or greater than that of known ascorbic acid derivatives, that is, 2-O-glyceryl ascorbic acid and 3-O-glyceryl ascorbic acid.

Test Example 2 [Stability test]

With a dilute potassium hydroxide aqueous solution, 1% aqueous solution of each test sample was adjusted to pH 6, placed in a 50 mL screw tube, sealed, and stored at 50°C for 4 weeks, and then subjected to HPLC measurement (using a liquid chromatograph manufactured by Shimadzu Corporation) to determine the residual rate from the peak area. The residual rate, odor, and coloring degree were evaluated according to the following methods and criteria. The results are shown in Tables 12 and 13.

Residual rate:

◎: 90% or more
○: 80% or more, less than 90%
Δ: 60% or more, less than 80%
×: 20% or more, less than 60%
××: Less than 20%

Odor: Evaluation was made by 10 panelists according to the following criteria.

3: Almost odorless.
2: There is a slight strange odor.
1: A strong odor is detected.

Based on the evaluation results, the following classification was made:

◎: Total score of 10 panelists is 25 or more
○: Total score of 10 panelists is 20-24
△: Total score of 10 panelists is 16-19
×: The total score of 10 panelists is 15 or less

Coloring: Evaluation was made by 10 panelists according to the following criteria.

3: Almost no change compared to immediately after preparation.
2: Color changes compared to immediately after preparation.
1: Strongly colored compared to immediately after preparation.

Based on the evaluation results, the following classification was made:

○: Total score of 10 panelists is 25 or more
△: Total score of 10 panelists is 16-24
×: The total score of 10 panelists is 15 or less

[Table 12]

| Example Number | Ascorbic acid derivatives or salts thereof | | initial | Two weeks later | Four weeks later |
|---|---|---|---|---|---|
| Comparison 1 | Ascorbic acid | Residual rate | ◎ | Δ | × |
| | | Odor | ○ | Δ | × |
| | | Coloring | ○ | Δ | × |
| Comparison 2 | 3-O-glyceryl ascorbic acid | Residual rate | ◎ | ○ | Δ |
| | | Odor | ○ | Δ | Δ |
| | | Coloring | ○ | Δ | Δ |
| 42 | 2-O-(2-hydroxyisobutyl) ascorbic acid (Synthesis Example 1) | Residual rate | ◎ | ◎ | ◎ |
| | | Odor | ○ | ○ | ○ |
| | | Coloring | ○ | ○ | ○ |
| 43 | 3-O-(2-hydroxyisobutyl) ascorbic acid (Synthesis Example 2) | Residual rate | ◎ | ◎ | ◎ |
| | | Odor | ○ | ○ | ○ |
| | | Coloring | ○ | ○ | ○ |
| 44 | 2-O-(2-hydroxyisobutyl)-3-O-dodecyl ascorbic acid (Synthesis Example 8) | Residual rate | ◎ | ◎ | ◎ |
| | | Odor | ○ | ○ | ○ |
| | | Coloring | ○ | ○ | ○ |
| 45 | 2-O-Tetradecyl-3-O-(2-hydroxyisobutyl) ascorbic acid (Synthesis Example 24) | Residual rate | ◎ | ◎ | ◎ |
| | | Odor | ○ | ○ | ○ |
| | | Coloring | ○ | ○ | ○ |
| 46 | 2,3-Di-O-(2-hydroxyisobutyl) ascorbic acid (Synthesis Example 17) | Residual rate | ◎ | ◎ | ◎ |
| | | Odor | ○ | ○ | ○ |
| | | Coloring | ○ | ○ | ○ |
| 47 | 2-O-(2-hydroxyisobutyl)-3-O-(2-hydroxy-3-O-butyl) ascorbic acid (Synthesis Example 14) | Residual rate | ◎ | ◎ | ◎ |
| | | Odor | ◎ | ◎ | ○ |
| | | Coloring | ○ | ○ | ○ |
| 48 | 2-O-(2-hydroxy-3-O-dodecyl)-3-O-(2-hydroxyisobutyl) ascorbic acid (Synthesis Example 31) | Residual rate | ◎ | ◎ | ◎ |
| | | Odor | ◎ | ◎ | ○ |
| | | Coloring | ○ | ○ | ○ |

[Table 13]

| Example Number | Ascorbic acid derivatives or salts thereof | | initial | Two weeks later | Four weeks later |
|---|---|---|---|---|---|
| 49 | 2-O-(2-hydroxyisobutyl)-3-O-(2-hydroxydecyl) ascorbic acid (Synthesis Example 36) | Residual rate | ◎ | ◎ | ◎ |
| | | Odor | ○ | ○ | ○ |
| | | Coloring | ◎ | ○ | ○ |
| 50 | 2-O-(2-hydroxyisobutyl)-3-O-(2-hydroxyhexadecyl) ascorbic acid (Synthesis Example 37) | Residual rate | ◎ | ◎ | ◎ |
| | | Odor | ◎ | ○ | ○ |
| | | Coloring | ○ | ○ | ○ |
| 51 | 2-O-(2-hydroxyisobutyl)-3-O-(2-hydroxyethylbenzene) ascorbic acid (Synthesis Example 38) | Residual rate | ◎ | ◎ | ◎ |
| | | Odor | ○ | ○ | ○ |
| | | Coloring | ◎ | ○ | ○ |
| 52 | 3-O-(2-hydroxyisobutyl)-2-O-(2-hydroxypropyl) ascorbic acid (Synthesis Example 39) | Residual rate | ◎ | ◎ | ◎ |
| | | Odor | ◎ | ○ | ○ |
| | | Coloring | ◎ | ○ | ○ |
| 53 | 3-O-(2-hydroxyisobutyl)-2-O-(2-hydroxybutyl) ascorbic acid (Synthesis Example 40) | Residual rate | ◎ | ◎ | ◎ |
| | | Odor | ○ | ○ | ○ |
| | | Coloring | ○ | ○ | ○ |
| 54 | 3-O-(2-hydroxyisobutyl)-2-O-(2-hydroxydecyl) ascorbic acid (Synthesis Example 41) | Residual rate | ◎ | ◎ | ◎ |
| | | Odor | ○ | ○ | ○ |
| | | Coloring | ○ | ○ | ○ |
| 55 | 3-O-(2-hydroxyisobutyl)-2-O-(2-hydroxyhexadecyl) ascorbic acid (Synthesis Example 42) | Residual rate | ◎ | ◎ | ◎ |
| | | Odor | ○ | ○ | ○ |
| | | Coloring | ○ | ○ | ○ |
| 56 | 3-O-(2-hydroxyisobutyl)-2-O-(2-hydroxyethylbenzene) ascorbic acid (Synthesis Example 43) | Residual rate | ◎ | ◎ | ◎ |
| | | Odor | ○ | ○ | ○ |
| | | Coloring | ○ | ○ | ○ |

The ascorbic acid derivatives of the present invention have superior stability over time when stored at 50°C compared to ascorbic acid, and show almost no odor generation or coloring, etc. That is, the results shown in Tables 12 and 13 show that the ascorbic acid derivatives of the present invention have the excellent properties inherent to ascorbic acid, such as excellent whitening effect, and also have improved stability over time, which was a problem with conventional ascorbic acid derivatives, and are therefore more suitable as ingredients for cosmetics.

Test Example 3 [Antioxidant test]

Normal human epidermal keratinocytes were seeded in a 96-well plate using KG2 medium to a cell density of 2.0 $\times 10^4$ cells/well. After 24 hours of pre-incubation, samples adjusted to a predetermined concentration with KG2 medium were added to each well. After 24 hours of culture and removal of the medium, the wells were washed with HBSS(-) and the ROS-reactive fluorescent probe DCFHDA was allowed to incorporate for 30 minutes. The wells were washed again with HBSS(-), treated with 0.2 mM $H_2O_2$, and cultured for 2 hours. The fluorescence intensity was measured, and the amount of ROS produced per unit protein was calculated by dividing the fluorescence intensity by the amount of protein quantified by the BCA method. These results are shown in Table 14.

The antioxidant effect when the sample was measured at a concentration of 10 mM or less was expressed as follows. The measurement was performed with N=4.

| | |
|---|---|
| <20% | : ± |
| 20-40% | : + |
| 40-70% | : ++ |
| 70-100% | : +++ |

[Table 14]

| Example Number | Ascorbic acid derivatives or salts thereof | Antioxidant Effects |
|---|---|---|
| Comparison | 2-O-glyceryl ascorbic acid | ± |
| Comparison | 3-O-glyceryl ascorbic acid | + |
| 57 | 2-O-(2-hydroxyisobutyl) ascorbic acid (Synthesis Example 1) | ++ |
| 58 | 3-O-(2-hydroxyisobutyl) ascorbic acid (Synthesis Example 2) | ++ |
| 59 | 2-O-(2-hydroxyisobutyl)-3-O-ethylascorbic acid (Synthesis Example 3) | ++ |
| 60 | 2-O-Ethyl-3-O-(2-hydroxyisobutyl) ascorbic acid (Synthesis Example 18) | ++ |

The results in Table 14 show that the ascorbic acid derivatives of the present invention have a superior antioxidant effect to known ascorbic acid derivatives or salts thereof, i.e., 2-O-glyceryl ascorbic acid and 3-O-glyceryl ascorbic acid.

Test Example 4 [Collagen production promoting effect ]

Normal human dermal fibroblasts were prepared in D-MEM containing 5% (v/v) fetal bovine serum to a cell density of 2.5 x $10^4$ cells/well, and pre-incubated on a 96-well plate for 24 hours. After removing the medium, samples prepared to a predetermined concentration in D-MEM containing 5% (v/v) fetal bovine serum were added to each well and cultured for 48 hours. After completion of the culture, the amount of free collagen in the supernatant was quantified by ELISA method. The measurement was performed with N=3.

The amount of collagen produced when the samples were measured at a concentration of 10 mmol/L or less was compared with that of the control group, and the results (% values when the control group is taken as 100%) are shown in Tables 15 to 17 based on the following criteria.

| | |
|---|---|
| <100% | : ± |
| 100-120% | : + |
| 120%< | : ++ |

[Table 15]

| Example Number | Ascorbic acid derivatives or salts thereof | Collagen production promoting effect |
|---|---|---|
| Comparison | 2-O-glyceryl ascorbic acid | + |
| Comparison | 3-O-glyceryl ascorbic acid | + |
| 61 | 2-O-(2-hydroxyisobutyl) ascorbic acid (Synthesis Example 1) | ++ |
| 62 | 3-O-(2-hydroxyisobutyl) ascorbic acid (Synthesis Example 2) | + |
| 63 | 2-O-(2-hydroxyisobutyl)-3-O-ethylascorbic acid (Synthesis Example 3) | ++ |
| 64 | 2-O-(2-hydroxyisobutyl)-3-O-butylascorbic acid (Synthesis Example 4) | ++ |
| 65 | 2-O-(2-hydroxyisobutyl)-3-O-hexyl ascorbic acid (Synthesis Example 5) | ++ |
| 66 | 2-O-(2-hydroxyisobutyl)-3-O-octyl ascorbic acid (Synthesis Example 6) | ++ |
| 67 | 2-O-(2-hydroxyisobutyl)-3-O-decyl ascorbic acid (Synthesis Example 7) | ++ |
| 68 | 2-O-(2-hydroxyisobutyl)-3-O-dodecyl ascorbic acid (Synthesis Example 8) | ++ |
| 69 | 2-O-(2-hydroxyisobutyl)-3-O-tetradecyl ascorbic acid (Synthesis Example 9) | ++ |

(continued)

| Example Number | Ascorbic acid derivatives or salts thereof | Collagen production promoting effect |
|---|---|---|
| 70 | 2-O-(2-hydroxyisobutyl)-3-O-allylascorbic acid (Synthesis Example 11) | ++ |
| 71 | 2-O-(2-hydroxyisobutyl)-3-O-benzyl ascorbic acid (Synthesis Example 12) | ++ |
| 72 | 2-O-(2-hydroxyisobutyl)-3-O-glyceryl ascorbic acid (Synthesis Example 13) | ++ |

[Table 16]

| Example Number | Ascorbic acid derivatives or salts thereof | Collagen production promoting effect |
|---|---|---|
| 73 | 2-O-(2-hydroxyisobutyl)-3-O-(2-hydroxy-3-O-butyl) ascorbic acid (Synthesis Example 14) | ++ |
| 74 | 2-O-(2-hydroxyisobutyl)-3-O-(2-hydroxy-3-O-ethylhexyl) ascorbic acid (Synthesis Example 15) | ++ |
| 75 | 2,3-Di-O-(2-hydroxyisobutyl) ascorbic acid (Synthesis Example 17) | ++ |
| 76 | 2-O-Ethyl-3-O-(2-hydroxyisobutyl) ascorbic acid (Synthesis Example 18) | ++ |
| 77 | 2-O-Butyl-3-O-(2-hydroxyisobutyl) ascorbic acid (Synthesis Example 19) | ++ |
| 78 | 2-O-Hexyl-3-O-(2-hydroxyisobutyl) ascorbic acid (Synthesis Example 20) | ++ |
| 79 | 2-O-Octyl-3-O-(2-hydroxyisobutyl) ascorbic acid (Synthesis Example 21) | ++ |
| 80 | 2-O-dodecyl-3-O-(2-hydroxyisobutyl) ascorbic acid (Synthesis Example 23) | ++ |
| 81 | 2-O-Tetradecyl-3-O-(2-hydroxyisobutyl) ascorbic acid (Synthesis Example 24) | ++ |
| 82 | 2-O-Hexadecyl-3-O-(2-hydroxyisobutyl) ascorbic acid (Synthesis Example 25) | ++ |
| 83 | 2-O-Allyl-3-O-(2-hydroxyisobutyl) ascorbic acid (Synthesis Example 26) | ++ |
| 84 | 2-O-benzyl-3-O-(2-hydroxyisobutyl) ascorbic acid (Synthesis Example 27) | ++ |
| 85 | 2-O-(2-hydroxy-3-O-butyl)-3-O-(2-hydroxyisobutyl) ascorbic acid (Synthesis Example 29) | ++ |
| 86 | 2-O-(2-hydroxy-3-O-dodecyl)-3-O-(2-hydroxyisobutyl) ascorbic acid (Synthesis Example 31) | ++ |
| 87 | 2-O-(2-hydroxyisobutyl)-3-O-propylascorbic acid (Synthesis Example 32) | ++ |
| 88 | 2-O-Propyl-3-O-(2-hydroxyisobutyl) ascorbic acid (Synthesis Example 33) | ++ |

[Table 17]

| Example Number | Ascorbic acid derivatives or salts thereof | Collagen production promoting effect |
|---|---|---|
| 89 | 2 -O-(2-hydroxyisobutyl)-3-O-(2-hydroxypropyl) ascorbic acid (Synthesis Example 34) | + |

(continued)

| Example Number | Ascorbic acid derivatives or salts thereof | Collagen production promoting effect |
|---|---|---|
| 90 | 2-O-(2-hydroxyisobutyl)-3-O-(2-hydroxybutyl) ascorbic acid (Synthesis Example 35) | ++ |
| 91 | 2-O-(2-hydroxyisobutyl)-3-O-(2-hydroxydecyl) ascorbic acid (Synthesis Example 36) | + |
| 92 | 2-O-(2-hydroxyisobutyl)-3-O-(2-hydroxyhexadecyl) ascorbic acid (Synthesis Example 37) | + |
| 93 | 2-O-(2-hydroxyisobutyl)-3-O-(2-hydroxyethylbenzene) ascorbic acid (Synthesis Example 38) | ++ |
| 94 | 3-O-(2-hydroxyisobutyl)-2-O-(2-hydroxypropyl) ascorbic acid (Synthesis Example 39) | + |
| 95 | 3-O-(2-hydroxyisobutyl)-2-O-(2-hydroxybutyl) ascorbic acid (Synthesis Example 40) | ++ |
| 96 | 3-O-(2-hydroxyisobutyl)-2-O-(2-hydroxydecyl) ascorbic acid (Synthesis Example 41) | + |
| 97 | 3-O-(2-hydroxyisobutyl)-2-O-(2-hydroxyhexadecyl) ascorbic acid (Synthesis Example 42) | + |
| 98 | 3-O-(2-hydroxyisobutyl)-2-O-(2-hydroxyethylbenzene) ascorbic acid (Synthesis Example 43) | ++ |

The results in Tables 15 to 17 clearly show that the ascorbic acid derivatives of the present invention have a collagen production promoting effect equal to or greater than that of known ascorbic acid derivatives or salts thereof, i.e., 2-O-glyceryl ascorbic acid and 3-O-glyceryl ascorbic acid.

Test Example 5 [Hyaluronic acid production promoting effect ]

Normal human dermal fibroblasts (NHDF) were prepared in D-MEM containing 5% (v/v) fetal bovine serum to a cell density of 2.5 $\times$ $10^4$ cells/well, and then pre-incubated on a 96-well plate for 24 hours. After removing the medium, samples prepared in serum-free D-MEM were added to each well and cultured for 48 hours. After the culture was completed, the amount of hyaluronic acid in the supernatant was quantified by ELISA method. The measurement was performed with N=3.

The amount of hyaluronic acid produced when the samples were measured at a concentration of 10 mmol/L or less was compared with that of the control group, and the results (% values when the control group is taken as 100%) are shown in Tables 18 to 20 based on the following criteria.

<100% : ±
100-120% : +
120-150% : ++
150 %< : +++

[Table 18]

| Example Number | Ascorbic acid derivatives or salts thereof | Hyaluronic acid production promoting effect |
|---|---|---|
| Comparison | 2-O-glyceryl ascorbic acid | + |
| Comparison | 3-O-glyceryl ascorbic acid | + |
| 99 | 2-O-(2-hydroxyisobutyl) ascorbic acid (Synthesis Example 1) | + |
| 100 | 3-O-(2-hydroxyisobutyl) ascorbic acid (Synthesis Example 2) | + |

(continued)

| Example Number | Ascorbic acid derivatives or salts thereof | Hyaluronic acid production promoting effect |
|---|---|---|
| 101 | 2-O-(2-hydroxyisobutyl)-3-O-dodecyl ascorbic acid (Synthesis Example 8) | +++ |
| 102 | 2-O-(2-hydroxyisobutyl)-3-O-tetradecyl ascorbic acid (Synthesis Example 9) | +++ |
| 103 | 2-O-(2-hydroxyisobutyl)-3-O-allylascorbic acid (Synthesis Example 11) | ++ |
| 104 | 2-O-(2-hydroxyisobutyl)-3-O-benzyl ascorbic acid (Synthesis Example 12) | +++ |
| 105 | 2-O-(2-hydroxyisobutyl)-3-O-glyceryl ascorbic acid (Synthesis Example 13) | ++ |
| 106 | 2-O-(2-hydroxyisobutyl)-3-O-(2-hydroxy-3-O-butyl) ascorbic acid (Synthesis Example 14) | ++ |
| 107 | 2-O-(2-hydroxyisobutyl)-3-O-(2-hydroxy-3-O-dodecyl) ascorbic acid (Synthesis Example 16) | ++ |
| 108 | 2,3-Di-O-(2-hydroxyisobutyl) ascorbic acid (Synthesis Example 17) | ++ |

[Table 19]

| Example Number | Ascorbic acid derivatives or salts thereof | Hyaluronic acid production promoting effect |
|---|---|---|
| 109 | 2-O-Ethyl-3-O-(2-hydroxyisobutyl) ascorbic acid (Synthesis Example 18) | ++ |
| 110 | 2-O-Octyl-3-O-(2-hydroxyisobutyl) ascorbic acid (Synthesis Example 21) | +++ |
| 111 | 2-O-dodecyl-3-O-(2-hydroxyisobutyl) ascorbic acid (Synthesis Example 23) | ++ |
| 112 | 2-O-Tetradecyl-3-O-(2-hydroxyisobutyl) ascorbic acid (Synthesis Example 24) | ++ |
| 113 | 2-O-Hexadecyl-3-O-(2-hydroxyisobutyl) ascorbic acid (Synthesis Example 25) | ++ |
| 114 | 2-O-Allyl-3-O-(2-hydroxyisobutyl) ascorbic acid (Synthesis Example 26) | ++ |
| 115 | 2-O-benzyl-3-O-(2-hydroxyisobutyl) ascorbic acid (Synthesis Example 27) | +++ |
| 116 | 2-O-Glyceryl-3-O-(2-hydroxyisobutyl) ascorbic acid (Synthesis Example 28) | ++ |
| 117 | 2-O-(2-hydroxy-3-O-ethylhexyl)-3-O-(2-hydroxyisobutyl) ascorbic acid (Synthesis Example 30) | +++ |
| 118 | 2-O-(2-hydroxy-3-O-dodecyl)-3-O-(2-hydroxyisobutyl) ascorbic acid (Synthesis Example 31) | ++ |
| 119 | 2-O-(2-hydroxyisobutyl)-3-O-propyl ascorbic acid (Synthesis Example 32) | ++ |
| 120 | 2-O-Propyl-3-O-(2-hydroxyisobutyl) ascorbic acid (Synthesis Example 33) | ++ |

[Table 20]

| Example Number | Ascorbic acid derivatives or salts thereof | Hyaluronic acid production promoting effect |
|---|---|---|
| 121 | 2-O-(2-hydroxyisobutyl)-3-O-(2-hydroxypropyl) ascorbic acid (Synthesis Example 34) | ++ |
| 122 | 2-O-(2-hydroxyisobutyl)-3-O-(2-hydroxybutyl) ascorbic acid (Synthesis Example 35) | ++ |
| 123 | 2-O-(2-hydroxyisobutyl)-3-O-(2-hydroxydecyl) ascorbic acid (Synthesis Example 36) | ++ |
| 124 | 2-O-(2-hydroxyisobutyl)-8-O-(2-hydroxyhexadecyl) ascorbic acid (Synthesis Example 37) | ++ |
| 125 | 2-O-(2-hydroxyisobutyl)-3-O-(2-hydroxyethylbenzene) ascorbic acid (Synthesis Example 38) | ++ |
| 126 | 3-O-(2-hydroxyisobutyl)-2-O-(2-hydroxypropyl) ascorbic acid (Synthesis Example 39) | +++ |
| 127 | 3-O-(2-hydroxyisobutyl)-2-O-(2-hydroxybutyl) ascorbic acid (Synthesis Example 40) | ++ |
| 128 | 3-O-(2-hydroxyisobutyl)-2-O-(2-hydroxydecyl) ascorbic acid (Synthesis Example 41) | ++ |
| 129 | 3-O-(2-hydroxyisobutyl)-2-O-(2-hydroxyhexadecyl) ascorbic acid (Synthesis Example 42) | +++ |
| 130 | 3-O-(2-hydroxyisobutyl)-2-O-(2-hydroxyethylbenzene) ascorbic acid (Synthesis Example 43) | ++ |

The results in Tables 18 to 20 clearly show that the ascorbic acid derivatives of the present invention have a hyaluronic acid production promoting effect equal to or greater than that of known ascorbic acid derivatives such as 2-O-glyceryl ascorbic acid.

Example 131 Cream

The oil phase ingredients (1) to (5) and the aqueous phase ingredients (6) to (10) shown in Table 21 were each heated to 70°C and dissolved to prepare an oil phase and an aqueous phase, respectively. The oil phase was then added to the aqueous phase and pre-emulsified, and the mixture was uniformly emulsified using a homomixer. The mixture was then cooled to room temperature while stirring thoroughly to prepare a cream with excellent whitening effects. In Table 22 and subsequent tables, the amounts are expressed in parts by mass.

[Table 21]

| No | Ingredient Name | Formulation amount |
|---|---|---|
| 1 | Squalane | 9.0 |
| 2 | Vaseline | 6.0 |
| 3 | Stearyl alcohol | 5.0 |
| 4 | Polyoxyethylene (25) cetyl ether | 2.5 |
| 5 | Glyceryl Monostearate | 1.5 |
| 6 | 2-O-benzyl-3-O-(2-hydroxyisobutyl) ascorbic acid (Synthesis Example 27) | 2.0 |
| 7 | Glycerin | 6.0 |
| 8 | Preservatives | Appropriate amount |
| 9 | pH adjuster | Appropriate amount |

(continued)

| No | Ingredient Name | Formulation amount |
|---|---|---|
| 10 | Purified water | Remainder* |
| * The amount required to make the total blended amount 100 parts by mass. The same applies to the following tables. | | |

Example 132 Milky lotion

The oil phase ingredients (1) to (9) and the aqueous phase ingredients (10) to (13) shown in Table 22 were each heated to 70°C and dissolved to prepare an oil phase and an aqueous phase, respectively. The oil phase was then added to the aqueous phase and pre-emulsified, and the mixture was uniformly emulsified using a homomixer. The mixture was then cooled to room temperature while stirring thoroughly to prepare a milky lotion with excellent whitening effects.

[Table 22]

| No | Ingredient Name | Formulation amount |
|---|---|---|
| 1 | Isostearyl palmitate | 5.0 |
| 2 | Jojoba oil | 2.0 |
| 3 | Dimethylpolysiloxane | 2.0 |
| 4 | Cetanol | 1.0 |
| 5 | Stearic acid | 1.5 |
| 6 | Beeswax | 2.5 |
| 7 | Paraffin wax | 2.5 |
| 8 | Polyoxyethylene (20) sorbitan monostearate | 1.2 |
| 9 | Polyoxyethylene (40) sorbitol tetraoleate | 1.5 |
| 10 | Propylene glycol | 10.0 |
| 11 | 2-O-(2-hydroxyisobutyl)-3-O-glyceryl ascorbic acid (Synthesis Example 13) | 3.0 |
| 12 | Preservatives | Appropriate amount |
| 13 | Purified water | Remainder |

Example 133 Milky lotion

The oil phase ingredients (5) to (10) and the aqueous phase ingredients (1) to (4) and (11) to (12) in the compositions shown in Table 23 were each heated to 70°C and dissolved to prepare an oil phase and an aqueous phase, respectively. The oil phase was then added to the aqueous phase and pre-emulsified, and the mixture was uniformly emulsified using a homomixer. The mixture was then cooled to room temperature while stirring thoroughly to prepare a milky lotion with excellent whitening effects.

[Table 23]

| No | Ingredient Name | Formulation amount |
|---|---|---|
| 1 | Dipropylene glycol | 5.0 |
| 2 | 2-O-Octyl-3-O-(2-hydroxyisobutyl) ascorbic acid (Synthesis Example 21) | 1.0 |
| 3 | Sorbitan sesquioleate | 4.0 |
| 4 | Polyoxyethylene (20) sorbitan monooleate | 1.0 |
| 5 | Microcrystalline wax | 1.0 |
| 6 | Beeswax | 2.0 |
| 7 | Lanolin | 2.0 |

(continued)

| No | Ingredient Name | Formulation amount |
|---|---|---|
| 8 | Liquid paraffin | 18.0 |
| 9 | Squalane | 12.0 |
| 10 | Fragrances | Appropriate amount |
| 11 | Preservatives | Appropriate amount |
| 12 | Purified water | Remainder |

Example 134 Cream

The oil phase ingredients (1) and (2) and the aqueous phase ingredients (3) to (10) shown in Table 24 are each heated to 70°C and dissolved to prepare an oil phase and an aqueous phase, respectively. Then the oil phase is added to the aqueous phase and pre-emulsified, and the mixture is uniformly emulsified using a homomixer. The mixture was then cooled to room temperature while stirring thoroughly to prepare a cream. This cream is used as a skin cosmetic with excellent whitening effects.

[Table 24]

| No | Ingredient Name | Formulation amount |
|---|---|---|
| 1 | Liquid Paraffin | 15.0 |
| 2 | Vaseline | 15.0 |
| 3 | Carboxyvinyl polymer | 0.1 |
| 4 | Xanthan gum | 0.1 |
| 5 | Polyoxyethylene (40) hydrogenated castor oil derivative | 3.0 |
| 6 | 2-O-(2-hydroxy-3-O-ethylhexyl)-3-O-(2-hydroxyisobutyl) ascorbic acid (Synthesis Example 30) | 5.0 |
| 7 | Sodium hydroxide | 0.05 |
| 8 | Fragrances | Appropriate amount |
| 9 | Preservatives | Appropriate amount |
| 10 | Purified water | Remainder |

Example 135 Skin lotion

A skin lotion can be prepared by thoroughly mixing the raw materials (1) to (6) shown in Table 25. This skin lotion contains 7% by weight of 2,3-di-O-(2-hydroxyisobutyl) ascorbic acid, and therefore has an excellent whitening effect.

[Table 25]

| No | Ingredient Name | Formulation amount |
|---|---|---|
| 1 | 2,3-Di-O-(2-hydroxyisobutyl) ascorbic acid (Synthesis Example 17) | 7.0 |
| 2 | Alcohol | 8.0 |
| 3 | Citric acid | 0.01 |
| 4 | Sodium citrate | 0.015 |
| 5 | Potassium glycyrrhizinate | 0.03 |
| 6 | Purified water | Remainder |

Example 136 Cream

The oil phases (1) to (6) and the aqueous phases (7) to (10) shown in Table 26 are each heated to 70°C and dissolved. The oil phase is added to the aqueous phase and pre-emulsified, then emulsified with a homomixer. The mixture is then cooled to room temperature while stirring thoroughly to prepare a cream. This cream contains 3% by weight of 3-O-(2-hydroxyisobutyl) ascorbic acid, and can be used as a skin cosmetic with excellent melanin production inhibitory effect and excellent whitening effect.

[Table 26]

| No | Ingredient Name | Formulation amount |
|---|---|---|
| 1 | Cetyl alcohol | 2.0 |
| 2 | Stearyl alcohol | 3.0 |
| 3 | Squalane | 7.5 |
| 4 | Glyceryl tri-2-ethylhexanoate | 7.5 |
| 5 | Methylpolysiloxane | 5.5 |
| 6 | 3-O-(2-hydroxyisobutyl) ascorbic acid (Synthesis Example 2) | 3.0 |
| 7 | 1,3-butylene glycol | 5.0 |
| 8 | Hydroxyethylcellulose | 0.2 |
| 9 | Preservatives | Appropriate amount |
| 10 | Purified water | Remainder |

Example 137 Cream

The oil phase ingredients (1) to (5) and the aqueous phase ingredients (6) to (10) shown in Table 27 were each heated to 70°C and dissolved to prepare an oil phase and an aqueous phase, respectively. The oil phase was then added to the aqueous phase and pre-emulsified, and the mixture was uniformly emulsified using a homomixer. The mixture was then cooled to room temperature while stirring thoroughly to prepare a cream with excellent whitening effects.

[Table 27]

| No | Ingredient Name | Formulation amount |
|---|---|---|
| 1 | Squalane | 9.0 |
| 2 | Vaseline | 6.0 |
| 3 | Stearyl alcohol | 5.0 |
| 4 | Polyoxyethylene (25) cetyl ether | 2.5 |
| 5 | Glyceryl Monostearate | 1.5 |
| 6 | 3-O-(2-hydroxyisobutyl)-2-O-(2-hydroxypropyl) ascorbic acid (Synthesis Example 39) | 2.0 |
| 7 | Glycerin | 6.0 |
| 8 | Preservatives | Appropriate amount |
| 9 | pH adjuster | Appropriate amount |
| 10 | Purified water | Remainder |

Example 138 Milky lotion

The oil phase ingredients (1) to (9) and the aqueous phase ingredients (10) to (13) shown in Table 28 were each heated to 70°C and dissolved to prepare an oil phase and an aqueous phase, respectively. The oil phase is then added to the aqueous phase and pre-emulsified, and the mixture was uniformly emulsified using a homomixer. The mixture is then cooled to room temperature while stirring thoroughly to prepare a milky lotion with excellent whitening effects.

[Table 28]

| No | Ingredient Name | Formulation amount |
|---|---|---|
| 1 | Isostearyl palmitate | 5.0 |
| 2 | Jojoba oil | 2.0 |
| 3 | Dimethylpolysiloxane | 2.0 |
| 4 | Cetanol | 1.0 |
| 5 | Stearic acid | 1.5 |
| 6 | Beeswax | 2.5 |
| 7 | Paraffin wax | 2.5 |
| 8 | Polyoxyethylene (20) sorbitan monostearate | 1.2 |
| 9 | Polyoxyethylene (40) sorbitol tetraoleate | 1.5 |
| 10 | Propylene Glycol | 10.0 |
| 11 | 3-O-(2-hydroxyisobutyl)-2-O-(2-hydroxyhexadecyl) ascorbic acid (Synthesis Example 42) | 3.0 |
| 12 | Preservatives | Appropriate amount |
| 13 | Purified water | Remainder |

Example 139 Milky lotion

The oil phase ingredients (5) to (10) and the aqueous phase ingredients (1) to (4) and (11) to (12) in the compositions shown in Table 29 were each heated to 70°C and dissolved to prepare an oil phase and an aqueous phase, respectively. The oil phase was then added to the aqueous phase and pre-emulsified, and the mixture was uniformly emulsified using a homomixer. The mixture is then cooled to room temperature while stirring thoroughly to prepare a milky lotion with excellent whitening effects.

[Table 29]

| No | Ingredient Name | Formulation amount |
|---|---|---|
| 1 | Dipropylene glycol | 5.0 |
| 2 | 2-O-(2-hydroxyisobutyl)-3-O-(2-hydroxypropyl) ascorbic acid (Synthesis Example 34) | 1.0 |
| 3 | Sorbitan sesquioleate | 4.0 |
| 4 | Polyoxyethylene (20) sorbitan monooleate | 1.0 |
| 5 | Microcrystalline wax | 1.0 |
| 6 | Beeswax | 2.0 |
| 7 | Lanolin | 2.0 |
| 8 | Liquid paraffin | 18.0 |
| 9 | Squalane | 12.0 |
| 10 | Fragrances | Appropriate amount |
| 11 | Preservatives | Appropriate amount |
| 12 | Purified water | Remainder |

Example 140 Cream

The oil phase ingredients (1) and (2) and the aqueous phase ingredients (3) to (10) in the composition shown in Table 30 are each heated to 70°C and dissolved to prepare an oil phase and an aqueous phase, respectively, and then the oil phase is added to the aqueous phase and pre-emulsified, and the mixture is uniformly emulsified using a homomixer. The mixture is then cooled to room temperature while stirring thoroughly to prepare a cream. This cream is used as a skin

cosmetic with excellent whitening effects.

[Table 30]

| No | Ingredient Name | Formulation amount |
|---|---|---|
| 1 | Liquid paraffin | 15.0 |
| 2 | Vaseline | 15.0 |
| 3 | Carboxyvinyl polymer | 0.1 |
| 4 | Xanthan gum | 0.1 |
| 5 | Polyoxyethylene (40) hydrogenated castor oil derivative | 3.0 |
| 6 | 3-O-(2-hydroxyisobutyl)-2-O-(2-hydroxybutyl) ascorbic acid (Synthesis Example 40) | 5.0 |
| 7 | Sodium hydroxide | 0.05 |
| 8 | Fragrances | Appropriate amount |
| 9 | Preservatives | Appropriate amount |
| 10 | Purified water | Remainder |

Example 141 Skin lotion

A skin lotion is prepared by thoroughly mixing the raw materials (1) to (6) in the composition shown in Table 31. This skin lotion contains 7% by mass of 2-O-(2-hydroxyisobutyl)-3-O-(2-hydroxybutyl) ascorbic acid, and therefore has an excellent whitening effect.

[Table 31]

| No | Ingredient Name | Formulation amount |
|---|---|---|
| 1 | 2-O-(2-hydroxyisobutyl)-3-O-(2-hydroxybutyl) ascorbic acid (Synthesis Example 35) | 7.0 |
| 2 | Alcohol | 8.0 |
| 3 | Citric acid | 0.01 |
| 4 | Sodium citrate | 0.015 |
| 5 | Potassium glycyrrhizinate | 0.03 |
| 6 | Purified water | Remainder |

Example 142 Cream

The oil phases (1) to (6) and the aqueous phases (7) to (10) in the composition shown in Table 32 are each heated to 70°C and dissolved. The oil phase is added to the aqueous phase and pre-emulsified, and then emulsified with a homomixer. The mixture is then cooled to room temperature while stirring thoroughly to prepare a cream. This cream contains 3% by mass of 2-O-(2-hydroxyisobutyl)-3-O-(2-hydroxydecyl) ascorbic acid, and can be used as a skin cosmetic having excellent melanin production inhibitory effect and excellent whitening effect.

[Table 32]

| No | Ingredient Name | Formulation amount |
|---|---|---|
| 1 | Cetyl alcohol | 2.0 |
| 2 | Stearyl alcohol | 3.0 |
| 3 | Squalane | 7.5 |
| 4 | Glyceryl tri-2-ethylhexanoate | 7.5 |
| 5 | Methylpolysiloxane | 5.5 |
| 6 | 2-O-(2-hydroxyisobutyl)-3-O-(2-hydroxydecyl) ascorbic acid (Synthesis Example 36) | 3.0 |

(continued)

| No | Ingredient Name | Formulation amount |
|---|---|---|
| 7 | 1,3-Butylene glycol | 5.0 |
| 8 | Hydroxyethylcellulose | 0.2 |
| 9 | Preservatives | Appropriate amount |
| 10 | Purified water | Remainder |

## Claims

**1.** An ascorbic acid derivative or a salt thereof, **characterized by** being represented by the following general formula (I).

[Chemical formula 1]

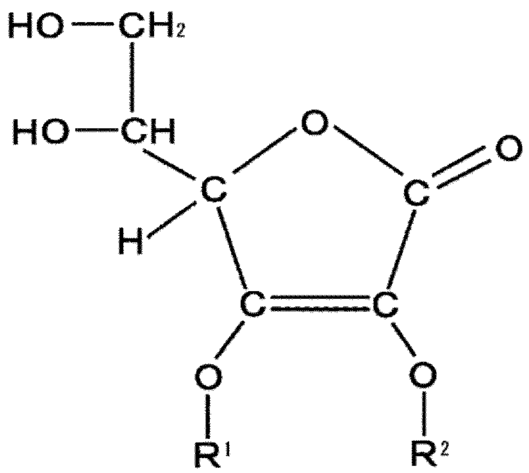

(I)

[wherein $R^1$ and $R^2$ are H, an alkyl group having 1 to 22 carbon atoms, an alkenyl group having 2 to 22 carbon atoms, a benzyl group, $R^3$ -O-$CH_2$ - CH(OH)-$CH_2$-, $R^3$-O-$CH_2$ -CH($CH_2$OH)-, $R^3$-CH($CH_2$OH)-, $R^3$-CH(OH)-$CH_2$-, HO-C$(CH_3)_2$-$CH_2$-, or HO-$CH_2$-C$(CH_3)_2$-, and $R^3$ is H, an alkyl group having 1 to 22 carbon atoms, an alkenyl group having 2 to 22 carbon atoms, or a phenyl group, provided that at least one of $R^1$ and $R^2$ is HO-C$(CH_3)_2$ -$CH_2$ - or HO-$CH_2$-C$(CH_3)_2$-.]

**2.** The ascorbic acid derivative or a salt thereof according to claim 1, wherein, in the general formula (I), one of $R^1$ and $R^2$ is HO-C$(CH_3)_2$-$CH_2$-or HO-$CH_2$-C$(CH_3)_2$-, and the other of $R^1$ and $R^2$ is an alkyl group having 2 to 20 carbon atoms or a branched alkylglyceryl group having 6 to 20 carbon atoms.

**3.** The ascorbic acid derivative or a salt thereof according to claim 1, wherein, in the general formula (I), one of $R^1$ and $R^2$ is HO-C$(CH_3)_2$-$CH_2$-or HO-$CH_2$ -C$(CH_3)_2$- and the other of $R^1$ and $R^2$ is a benzyl group.

**4.** The ascorbic acid derivative or a salt thereof according to claim 1, wherein, in the general formula (I), one of $R^1$ and $R^2$ is HO-C$(CH_3)_2$-$CH_2$-or HO-$CH_2$ -C$(CH_3)_2$- and the other of $R^1$ and $R^2$ is $R^3$-O-$CH_2$-CH(OH)-$CH_2$- or $R^3$-O-$CH_2$-CH($CH_2$OH)-.

**5.** The ascorbic acid derivative or a salt thereof according to claim 1, wherein, in the general formula (I), one of $R^1$ and $R^2$ is HO-C$(CH_3)_2$-$CH_2$-or HO-$CH_2$ -C$(CH_3)_2$- and the other of $R^1$ and $R^2$ is H, alkyl or alkenyl, and the total number of carbon atoms in $R^1$ and $R^2$ is 8 or less, or the other of $R^1$ and $R^2$ is $R^3$-O-$CH_2$-CH(OH)-$CH_2$-, $R^3$-O-$CH_2$-CH($CH_2$OH)-, HO-C$(CH_3)_2$-$CH_2$-or HO-$CH_2$ -C$(CH_3)_2$-, and the number of carbon atoms in $R^3$ is 4 or less.

**6.** The ascorbic acid derivative or a salt thereof according to claim 1, wherein, in the general formula (I), one of $R^1$ and $R^2$

is HO-$C(CH_3)_2$-$CH_2$-or HO-$CH_2$-$C(CH_3)_2$-, and the other of $R^1$ and $R^2$ is $R^3$-CH($CH_2$OH)- or $R^3$-CH(OH)-$CH_2$-.

**7.** A cosmetic which contains the ascorbic acid derivative or a salt thereof according to any one of claims 1-6.

**8.** A cosmetic in which the ascorbic acid derivative or a salt thereof according to any one of claims 1-6 is incorporated in an amount of 1 to 20% by mass

## INTERNATIONAL SEARCH REPORT

International application No.
**PCT/JP2023/022484**

**A. CLASSIFICATION OF SUBJECT MATTER**

***C07D 307/62***(2006.01)i; ***A61K 8/67***(2006.01)i; ***A61Q 19/02***(2006.01)i
FI: C07D307/62 CSP; A61K8/67; A61Q19/02

According to International Patent Classification (IPC) or to both national classification and IPC

**B. FIELDS SEARCHED**

Minimum documentation searched (classification system followed by classification symbols)

C07D307/62; A61K8/67; A61Q19/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN), JSTPlus/JMEDPlus/JST 7580 (JDreamIII), CiNii

**C. DOCUMENTS CONSIDERED TO BE RELEVANT**

| Category* | Citation of document, with indication, where appropriate, of the relevant passages | Relevant to claim No. |
|---|---|---|
| A | WO 2009/025328 A1 (SEIWAKASEI CO., LTD.) 26 February 2009 (2009-02-26) entire text | 1-8 |
| A | JP 2005-060239 A (NONOGAWA SHOJI Y.K.) 10 March 2005 (2005-03-10) entire text | 1-8 |
| A | JP 2017-088526 A (SEIWAKASEI CO., LTD.) 25 May 2017 (2017-05-25) entire text | 1-8 |
| A | JP 2016-113394 A (SEIWAKASEI CO., LTD.) 23 June 2016 (2016-06-23) entire text | 1-8 |
| A | JP 2016-121099 A (SEIWAKASEI CO., LTD.) 07 July 2016 (2016-07-07) entire text | 1-8 |
| A | JP 1-228978 A (NIPPON HYPOX LABORATORIES INC.) 12 September 1989 (1989-09-12) entire text | 1-8 |
| P, X | JP 7267657 B1 (SEIWAKASEI CO., LTD.) 24 April 2023 (2023-04-24) entire text, all drawings | 1-8 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 July 2023** | **29 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/022484**

| Patent document cited in search report | Publication date (day/month/year) | Patent family member(s) | Publication date (day/month/year) |
|---|---|---|---|
| WO 2009/025328 A1 | 26 February 2009 | EP 2189452 A1<br>all pages | |
| JP 2005-060239 A | 10 March 2005 | (Family: none) | |
| JP 2017-088526 A | 25 May 2017 | (Family: none) | |
| JP 2016-113394 A | 23 June 2016 | (Family: none) | |
| JP 2016-121099 A | 07 July 2016 | (Family: none) | |
| JP 1-228978 A | 12 September 1989 | EP 411182 A1<br>all pages | |
| JP 7267657 B1 | 24 April 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62221611 A
- JP 2005060239 A
- JP 1228978 A
- JP 4681670 B
- JP 2022028125 A